(19) Europäisches Patentamt
European Patent Office
Office européen des brevets

(11) Publication number: 0 171 702
A1

# (12) EUROPEAN PATENT APPLICATION

(21) Application number: 85109577.8

(22) Date of filing: 30.07.85

(51) Int. Cl.[4]: C 07 D 265/36
C 07 D 413/12, A 61 K 31/535

(30) Priority: 03.08.84 GB 8419797

(43) Date of publication of application:
19.02.86 Bulletin 86/8

(84) Designated Contracting States:
BE CH DE FR GB IT LI NL

(71) Applicant: BEECHAM GROUP PLC
Beecham House Great West Road
Brentford Middlesex TW8 9BD(GB)

(72) Inventor: Duckworth, David Malcolm
44 Meadow Walk Walton-On-The-Hill
Tadworth Surrey, KT20 7UG(GB)

(72) Inventor: Jennings, Leslie John Arthur
2 Logmore Green Cottage Logmore Lane
Westcott Surrey, RH4 3JN(GB)

(74) Representative: Hardman, Carol Pauline et al,
Beecham Pharmaceuticals Great Burgh Yew Tree
Bottom Road
Epsom Surrey KT18 5XQ(GB)

(54) **Benzoxazinone derivatives, preparation and use.**

(57) An unsubstituted or substituted (aryl- or heterocyclyl- or aryloxy- or heterocyclyloxy-)$C_{2-3}$-alkanolamino-($C_{2-4}$alkyl- or $C_{2-3}$alkoxy)-benzoxazinone or a pharmaceutically acceptable salt thereof or ester thereof; a process for preparing such a compound, pharmaceutical compositions containing them and their use in medicine and agriculture.

EP 0 171 702 A1

Croydon Printing Company Ltd

# Novel Compounds

The invention is concerned with secondary amines which have anti-hyperglycaemic and/or anti-obesity activity, and/or positive inotropic activity, with processes for their preparation, pharmaceutical compositions containing them and their use in medicine and agriculture.

Secondary amines having a variety of pharmacological properties are already known. For example, U.S. Patent Specification No. 4 349 673 discloses certain secondary amines which can be used to treat, for example, glaucoma, asthma, and hypertension, and German Offenlegungsschrift No. 24 29 253 discloses secondary (and tertiary) amines which can be used to treat, for example, high blood pressure and circulatory disorders. These disclosures include secondary amines that are aryl- or aryloxy-$C_{2-7}$alkylamino-$C_2$alkanoloxy-benzoxazinone or -$C_{2-3}$alkanolbenzoxazinone derivatives.

We have now discovered a group of pharmacologically active secondary amines that are N-[(aryl or heterocyclyl or aryloxy or heterocyclyloxy)-$C_{2-3}$alkyl] -N-[benzoxazinonyl-($C_{2-4}$alkyl or $C_{2-3}$oxyalkyl)] amines having a hydroxy group, or functional derivative thereof, as a substituent of the carbon atom β to the nitrogen atom in the alkylene group directly attached to the aryl or heterocyclyl or aryloxy or heterocyclyloxy radical.

It will be understood that the terms 'aryl' and 'heterocyclyl' do not include benzoxazinonyl and that the terms 'aryloxy' and 'heterocyclyloxy' do not include benzoxazinoyloxy.

Accordingly, the present invention provides an unsubstituted or substituted (aryl- or heterocyclyl- or aryloxy- or heterocyclyloxy-)$C_{2-3}$-alkanolamino-($C_{2-4}$alkyl- or $C_{2-3}$alkoxy)-benzoxazinone or a pharmaceutically acceptable salt thereof or ester thereof.

The aryl group is suitably monocyclic and thus preferably the aryl or aryloxy group is a phenyl or phenoxy group which is unsubstituted or substituted.

The aryl or aryloxy groups may be substituted with up to three groups selected from: hydroxy or an ester thereof or an ether thereof, lower alkyloxy, lower alkenyloxy, halogen, $CF_3$, $CH_2OH$ or an ester thereof, $NH_2$, $NHR^t$, $NHCOOR^t$ or $CH_2SO_2R^t$ wherein $R^t$ represents a lower alkyl group.

The heterocyclyl group (and the heterocyclyl moiety in the heterocyclyloxy group) is suitably a substituted or unsubstituted fused bicyclic group comprising up to two, preferably one, hetero atoms; preferred heterocyclyl groups include those wherein a phenyl ring is fused to a five membered ring, the five membered ring containing the hetero atom or atoms; suitably the phenyl ring bears the substituents. Favoured substituents are, up to three of: lower alkyl, lower alkyloxy, halo-lower-alkyl, hydroxy, amino, carboxy,

lower alkoxy carbonyl and lower alkoxy carbonyl-lower alkyl. A preferred substituent is a lower alkyl group, preferably on the phenyl ring. Favoured hetero atoms are oxygen and nitrogen atoms.

The benzoxazinonyl radical may be linked to the alkyl or alkoxy radical in the 6- or 7-position and it may be unsubstituted or substituted. Where substitution occurs, this is preferably at the nitrogen atom. More especially, however, the nitrogen atom is unsubstituted. A favoured substituent is a lower alkyl or a lower alkyl carbonyl group. A preferred substituent is a lower alkyl group.

More particularly, the invention provides a compound of formula (A):

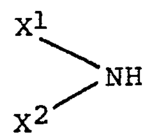

(A)

or a pharmaceutically acceptable salt thereof, wherein:

$X^1$ represents a 2-hydroxy-3-phenoxypropyl radical unsubstituted or substituted in the phenyl ring, or a 2-hydroxy-2-phenethyl radical unsubstituted or substituted in the phenyl ring or a 2-hydroxy-2-(benzofuranyl)ethyl radical or a 2-hydroxy-3-(indolyl)oxypropyl radical wherein a phenyl moiety in either of the benzofuranyl and indolyl moieties may be substituted or unsubstituted, and

$X^2$ represents a benzoxazinonylethyl, benzoxazinonylpropyl or benzoxazinonyloxyethyl radical unsubstituted or substituted in the benzoxazinonyl radical and/or lower alkyl-substituted in the alkyl chain.

The compounds have valuable pharmacological properties exhibiting anti-obesity and/or anti-hyperglycaemic activity, and/or positive inotropic activity.

Certain of the compounds have potent anti-obesity and anti-hyperglycaemic activity coupled with low cardiac stimulation activity, while other compounds show a marked positive inotropic activity.

Functional derivatives of the above hydroxy compounds, in which the hydroxy group is present in the form of an in vivo hydrolysable ester, also show the specified pharmacological activities.

A suitable substituent for the phenyl group in either of the benzofuranyl and indolyl moieties is $C_{1-6}$ alkyl. Suitably, the phenyl group in the benzofuranyl moiety is substituted in the 7- position, suitably with a $C_{1-4}$ alkyl group such as for example methyl or ethyl.

Suitably the phenyl group in the indolyl moiety is unsubstituted.

Thus, more particularly, the present invention provides a compound of the general formula (I):

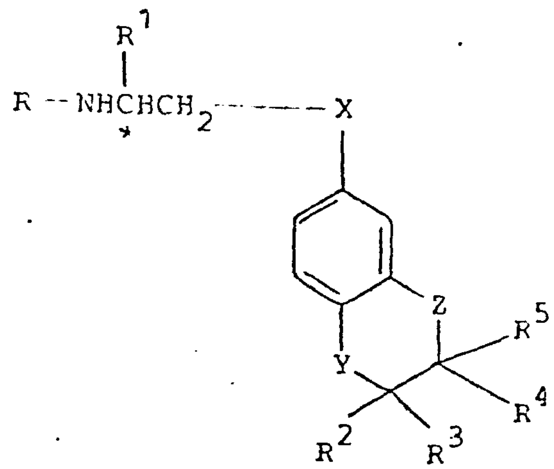

(I)

in which

R represents a 2-hydroxy-2-(benzofuranyl)ethyl moiety or a 2-hydroxy-2-(indolyl)oxypropyl moiety wherein the phenyl group in either of the two moieties may be substituted or unsubstituted; or a radical of the general formula (a) or (b):

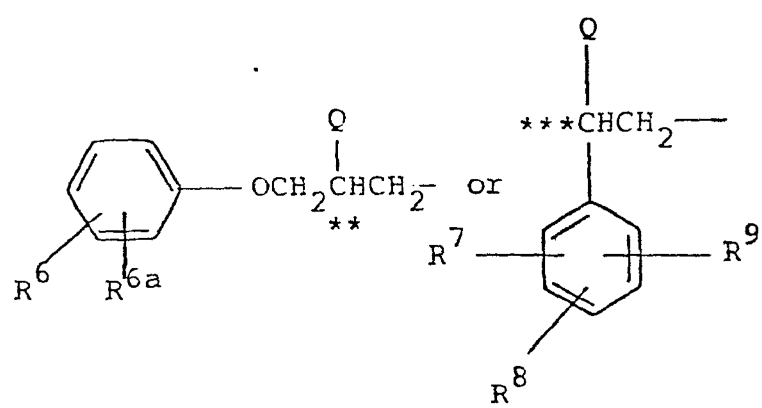

or

(a) (b)

$R^1$ represents a hydrogen atom or a lower alkyl group, $R^6$ and $R^{6a}$ each represent a hydrogen atom or a free hydroxy group or an ester thereof or a lower-alkyloxy group or a lower alkenyloxy group.

$R^7$ represents a hydrogen or halogen atom, a hydroxy group or an ester thereof or an ether thereof, or a group $CF_3$, $CH_2OH$ or an ester thereof, $NH_2$, $NHR^{11}$, $NHCOOR^{11}$, or $CH_2SO_2R^{11}$ in which $R^{11}$ represents an alkyl radical,

$R^8$ represents a hydrogen or halogen atom, or a hydroxy group or an ester thereof or an ether thereof,

$R^9$ represents a hydrogen or halogen atom,
Q represents a hydroxy group or an <u>in vivo</u> hydrolysable ester thereof, X represents a bond, a methylene group or an oxygen atom, and

Y represents an oxygen atom or a radical $NR^{10}$ in which $R^{10}$ represents a hydrogen atom or an alkyl group, and Z represents an oxygen atom or a radical $NR^{10}$ with the proviso that

when Y represents an oxygen atom, Z represents a radical $NR^{10}$, $R^2$ and $R^3$ each represents a hydrogen atom and $R^4$ and $R^5$ together represent an oxo group
or
when Y represents a radical $NR^{10}$, Z represents an oxygen atom, $R^2$ and $R^3$ together represent an oxo group and $R^4$ and $R^5$ each represents a hydrogen atom.

Preferably, the 2-hydroxy-2-(benzofuranyl)ethyl moiety is a 2-hydroxy-2-(benzofuran-2-yl)ethyl moiety.

Preferably, the 2-hydroxy-2-(indolyl)oxy propyl moiety is a 2-hydroxy-2-(indol-4-yl)oxypropyl moiety.

The invention also provides salts of compounds of the general formula (I), and more especially pharmaceutically acceptable salts thereof.

The compounds of the general formula (I) have, depending on the meaning of $R^1$, one or two asymmetric carbon atoms, marked with asterisks in the formula. These compounds may, therefore, exist in up to four stereoisomeric forms. The present invention encompasses all stereoisomers of the compounds of the general formula (I) whether free from other isomers or admixed with other isomers in any proportion, and thus includes, for instance, racemic mixtures of enantiomers.

The term 'halogen' refers to fluorine, chlorine, bromine and iodine.
Suitable esters are "in-vivo" hydrolysable esters.
When used herein in connection with alkyl, alkyloxy and alkenyloxy radicals, the term ''lower'' indicates that the radical or compound has from 1 to 6 carbon atoms, preferably 1 to 4 carbon atoms. The alkyl radical or the alkyl moiety in the alkoxy or alkenyloxy radical may be straight-or branched-chain, e.g. a methyl, ethyl, n-propyl, isopropyl, n-butyl, isobutyl or a tert-butyl group.

When used herein the term ''in-vivo'' hydrolysable ester'' of a hydroxy group relates to pharmaceutically acceptable ester groups which break down readily in the body to leave the free hydroxy group; any conventionally used acid may be used to form the ester although preferred esters are those formed with lower alkyl carboxylic acids.

Preferably, however, when any of the groups $R^6$, $R^{6a}$, $R^7$, $R^8$ or Q represent a hydroxy group, the

hydroxy group is present as a free hydroxy group. Similarly when $R^7$ represents $CH^2OH$.

When used herein the term ''ether'' of a hydroxy group relates to alkyl ethers, preferably lower alkyl, or aralkyl ethers, preferably benzyl.

There should especially be mentioned compounds of the general formula (I) in which R represents a 2-hydroxy-2-(7-lower alkyl benzofuran-2-yl)ethyl group or a 2-hydroxy-3-(indol-4-yl)oxypropyl group or a radical of general formula (a') or (b'):

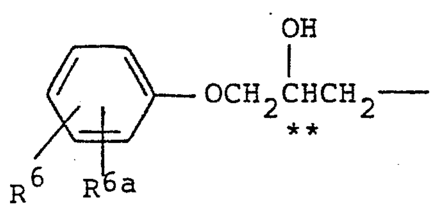

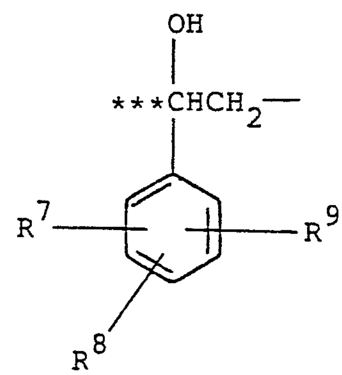

(a') or (b')

and $R^6$, $R^{6a}$, $R^7$, $R^8$ and $R^9$ are as defined in relation to formula (I).

Preferably $R^1$ in general formula (I) represents a methyl group.

Favourably, when R represents a radical of the formula (a), $R^6$ represents a free hydroxy group which may be in the <u>ortho</u>-, <u>meta</u>- or <u>para</u>- position and is especially a para-hydroxy substituent; and $R^{6a}$ represents a hydrogen atom.

Favourably, when R represents a radical of formula (a), $R^6$ and $R^{6a}$ each represent a hydroxyl group; preferably the two hydroxyl groups are in the <u>meta</u>- position.

Favourably, when R represents a radical of formula (a), $R^6$ represents a lower alkoxy group, especially a methoxy group, or a lower alkenyloxy group, especially an allyloxy group, and $R^{6a}$ represents a hydrogen atom; preferably the lower alkoxy and lower alkenyloxy groups are each in the <u>ortho</u>- position.

Favourably, when R represents a radical of formula (a), $R^6$ and $R^{6a}$ each represent a hydrogen atom.

More especially, the moiety of formula (a) is one of the following groups:

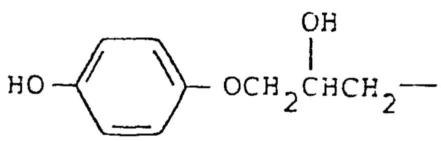 ,

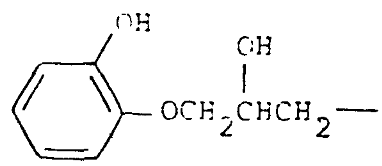

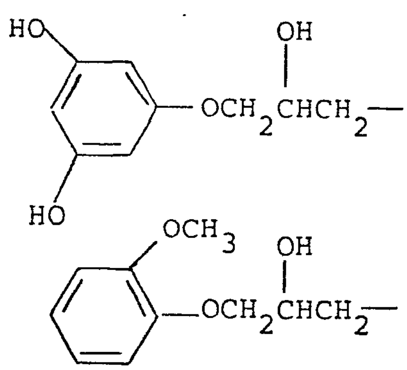 ,

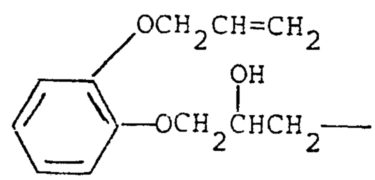

,

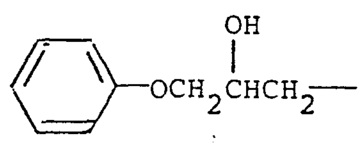

When R represents a radical of the formula (b), $R^7$ preferably represents a hydrogen or halogen atom, or a hydroxy, lower alkoxy, benzyloxy, $CF_3$, $CH_2OH$ or $NH_2$ group, $R^8$ preferably represents a hydrogen or chlorine

atom, or a hydroxy, lower alkoxy or benzyloxy radical, and R $^9$ preferably represents a hydrogen or chlorine atom.

Favourably, one of $R^7$, $R^8$ and $R^9$ represents a chlorine atom or a hydroxy group and the other two radicals each represent a hydrogen atom. In such cases, when a single substituent is present, it is preferably in the <u>meta</u>- or <u>para</u>- position.

Favourably, one of $R^7$, $R^8$ and $R^9$ represents a hydrogen atom and the other two radicals each represent a chlorine atom. Preferably, the chlorine atoms are present in the <u>meta</u>- and <u>para</u>- positions.

Favourably $R^7$, $R^8$ and $R^9$ each represent hydrogen.

A halogen atom represented by $R^8$ and/or $R^9$ is preferably a chlorine atom, and in the radical $R^7$ an alkyl radical $R^{11}$ is preferably a lower alkyl radical, especially a methyl group.

An ester of a hydroxy or hydroxy-containing group represented by $R^7$ or $R^8$ in formula (b) [and also by $R^6$ and $R^{6a}$ in formula (a)] is an ester of, for example, a lower alkyl carboxylic acid.

Preferably, however, the moiety of the formula (b) is one of the following groups:

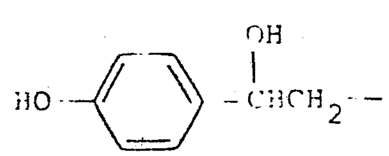

,

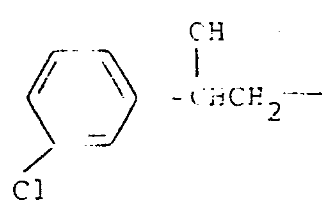

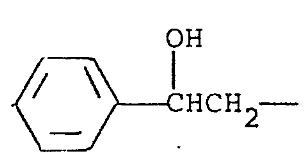

,

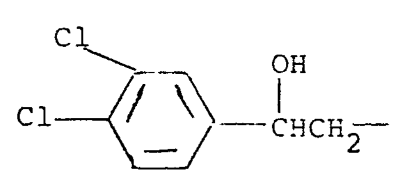

When $R^{10}$, present in radical Y or Z above, represents an alkyl radical it is preferably a lower alkyl radical. More especially, however, $R^{10}$ represents a hydrogen atom.

In a particularly preferred aspect the present invention provides a compound selected from the list consisting of:

7-[2-[2-(4-hydroxyphenyl)-2-hydroxyethylamino]-2-methylethoxy]-2H-1,4-benzoxazin-3-(4H)-one;

7-[2-[2-(3-chlorophenyl)-2-hydroxyethylamino]-2-methylethoxy]-2H-1,4-benzoxazin-3-(4H)-one;

7-[2-[3-(4-hydroxyphenoxy)-2-hydroxypropylamino]-2-methylethoxy]-2H-1,4-benzoxazin-3-(4H)-one;

6-[2-[3-(4-hydroxyphenoxy)-2-hydroxypropylamino]-2-methylethoxy]-2H-1,4-benzoxazin-3-(4H)-one;

6-[2-[3-(2-hydroxyphenoxy)-2-hydroxypropylamino]-2-methylethoxy]-2H-1,4-benzoxazin-3-(4H)-one;

6-[2-[2-(3-chlorophenyl)-2-hydroxyethylamino]-2-methylethoxy]-2H-1,4-benzoxazin-3-(4H)-one;

6-[2-[2-(3,4-dichlorophenyl)-2-hydroxyethylamino]-2-methylethoxy]-2H-1,4-benzoxazin-3-(4H)-one;

6-[2-[2-(3-chlorophenyl)-2-hydroxyethylamino]-2-methylethyl]-2H-1,4-benzoxazin-3-(4H)-one;

6-[2-[3-(2-hydroxyphenoxy)-2-hydroxypropylamino]-2-methylethyl]-2H-1,4-benzoxazin-3-(4H)-one;

6-[2-[2-phenyl-2-hydroxyethylamino]-2-methylethyl]-2H-1,4-benzoxazin-3-(4H)-one;

6-[2-[3-phenoxy-2-hydroxypropylamino]-2-methylethyl]-2H-1,4-benzoxazin-3-(4H)-one;

6-[2-[2-(3-chlorophenyl)-2-hydroxyethylamino]-2-methylethoxy]-4-methyl-2H-1,4-benzoxazin-3-(4H)-one;

6-[2-[2-(3-chlorophenyl)-2-hydroxyethylamino]-2-methylethyl]-4-methyl-2H-1,4-benzoxazin-3-(4H)-one;

6-[2-[3-(4-hydroxyphenoxy)-2-hydroxypropylamino]-2-methylethyl]-2H-1,4-benzoxazin-3-(4H)-one;

6-[2-[2-(4-hydroxyphenyl)-2-hydroxyethylamino]-2-methylethoxy]-2H-1,4-benzoxazin-3-(4H)-one;

6-[2-[3-(2-allyloxyphenoxy)-2-hydroxypropylamino]-2-methylethoxy]-2H-1,4-benzoxazin-3-(4H)-one;

6-[2-[3-(2-methoxyphenoxy)-2-hydroxypropylamino]-2-methylethoxy]-2H-1,4-benzoxazin-3-(4H)-one;

6-[2-(2-hydroxy-3-phenoxypropylamino)-2-methylethoxy]-2H-1,4-benzoxazin-3-(4H)-one;

6-[2-[2-hydroxy-3-(indol-4-yloxy)propylamino]-2-methyl-ethoxy]-2H-1,4-benzoxazin-3-(4H)-one;

6-[2-[2-(7-ethylbenzofuran-2-yl)-2-hydroxyethylamino]-2-methylethoxy]-2H-1,4-benzoxazin-3-(4H)-one; and

6-[2-[3-(3,5-dihydroxyphenoxy)-2-hydroxypropylamino]-2-methylethoxy]-2H-1,4-benzoxazin-3-(4H)-one; or a pharmaceutically acceptable salt thereof.

The absolute configuration of any compound of the general formula (I) may be determined by conventional X-ray crystallographic techniques.

Preferably the asymmetric carbon atoms have the following configurations:

*: the R-configuration,

**: the S-configuration,

***: the R-configuration.

The salts, particularly the pharmaceutically acceptable salts, of compounds of the invention are preferably acid addition salts; salt formation is possible for example with the nitrogen atom shown in the formula (I) and/or with the amino group $R^7$.

Acid addition salts may be, for example, salts with inorganic acids such, for example, as hydrochloric acid, hydrobromic acid, orthophosphoric acid or sulphuric acid, or organic acids such, for example as methanesulphonic acid, toluenesulphonic acid, acetic acid, propionic acid, lactic acid, citric acid, fumaric acid, malic acid, succinic acid, salicyclic acid or acetylsalicyclic acid.

The invention also provides a process for the preparation of a compound of the hereinbefore defined formula (A), or a pharmaceutically acceptable salt thereof, which process comprises: either

(a) reacting a compound of formula (B):

$$Y^1NH_2 \qquad (B)$$

wherein $Y^1$ represents either $X^1$ or $X^2$, and wherein $X^1$ and $X^2$ are as defined in relation to formula (I), with:

(i) a compound of formula (C):

$$Y^2\text{-}A \qquad (C)$$

wherein $Y^2$ represents either $X^1$ or $X^2$, as defined above, but does not represent $Y^1$, and A represents a leaving group; or

(ii) a compound of formula (D):

$$Y^3\text{-}A \qquad (D)$$

wherein $Y^3$ represents a group which is convertable, preferably by reduction, to a group of formula $Y^2$ after attachment to the nitrogen atom of formula (B) and A represents a leaving group; or

(b) reacting a compound of formula (E):

$$X^2NH_2 \qquad (E)$$

wherein $X^2$ is as defined above, with a compound of formula (F):

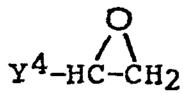

(F)

wherein $Y^4$ represents a group which when present

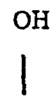

in the moiety $Y^4-CH-CH_2-$ the moiety represents $X^1$, or

(c) by reducing a compound of formula (G):

$$Y^4-CO-C{=}N-X^2 \qquad (G)$$

wherein $Y^4$ and $X^2$ are as defined above;
and thereafter, if necessary, carrying out one or more of the following steps:

(a) converting a compound of formula (A) to another compound of formula (A);

(b) converting any hydroxy group to an ester group thereof;

and if required, thereafter, forming a pharmaceutically acceptable salt of the compound so formed.

The compounds of formula (G) may be prepared from a compound of the hereinbefore defined formula (E) by reraction with a compound of formula (J):

$Y^4$-CO-CHO (J):

wherein $Y^4$ is as defined in relation to formula (F).

Preferably the compound of formula (G) above is not isolated but is reduced in-situ.

The invention also provides a process for the preparation of a compound of the general formula (I) or a pharmaceutically acceptable salt thereof, which comprises reacting a compound of the general formula (II):

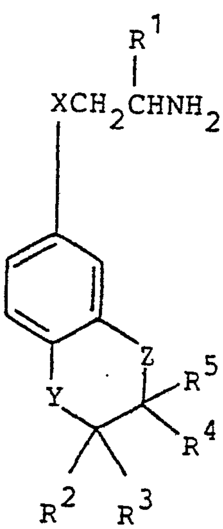

(II)

in which $R^1$, $R^2$, $R^3$, $R^4$, $R^5$, X, Y and Z are as defined in relation to formula (I) or a salt or reactive derivative thereof, with

(i) a compound of the general formula (III):

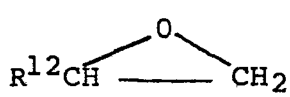

(III)

in which $R^{12}$ represents a benzofuranyl moiety (preferably a benzofuran-2-yl moiety) or an indolyloxy methylene moiety (preferably an (indol-4-yl) oxymethylene moiety) wherein the phenyl group in either of the two moieties may be substituted or unsubstituted, or $R^{12}$ represents a group of the general formula:

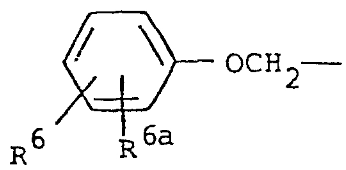

or

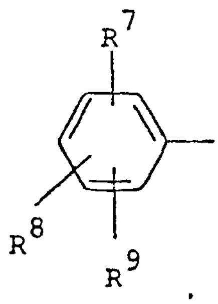

in which $R^6$, $R^{6a}$, $R^7$, $R^8$ and $R^9$ are as defined in relation to formula (I),
or

(ii) a compound of the general formula (IV):

$R^{12}CHCH_2A$ (IV)
|
OH

in which $R^{12}$ has the meaning given above and A represents a leaving group as hereinafter defined, or an ester thereof,

and, if desired, converting a resulting compound of the general formula (I) into another compound of the general formula (I) or into a pharmaceutically acceptable salt thereof.

A suitable substituent for phenyl group of the benzofuranyl or indolyl moieties is a $C_{1-6}$ alkyl group.

The reaction between compounds of the general formulae (II) and (III); or (E) and (F), is advantageously carried out in a protic solvent, e.g. a lower alkanol having at least 2 carbon atoms, at reflux, preferably in ethanol.

The reactions between compounds of the general formulae (II) and (IV); or (B) and (C), is advantageously carried out in dimethyl sulphoxide, for example at a temperature in the range of from 30 to 80°C, e.g. substantially 50°C and advantageously for a period of time of 1 to 4 days, e.g. about 3 days.

The invention further provides a process for the preparation of a compound of the general formula (I) or a pharmaceutically acceptable salt thereof, which comprises reacting a compound of the general formula (II) specified above, or a salt or reactive derivative thereof, with

(i) a compound of the general formula (V):

$$R^{12}COCHO \qquad (V)$$

in which $R^{12}$ is as defined in relation to formula (III), and subsequently reducing the resulting compound of the general formula (VI):

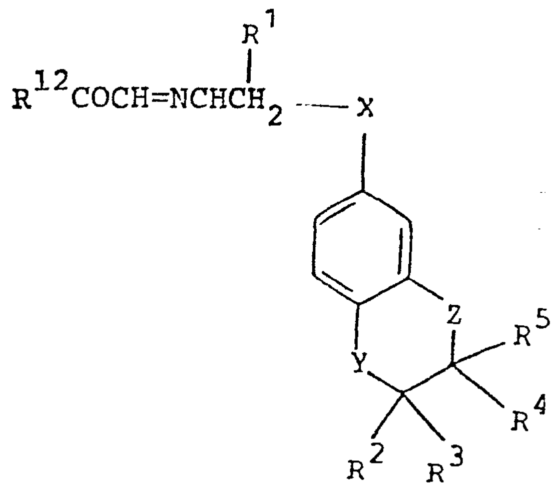

(VI)

in which $R^1$, $R^2$, $R^3$, $R^4$, $R^5$, X, Y and Z are as defined in relation to formula (I) and $R^{12}$ is as defined in relation to formula (III), or a salt thereof;

or

(ii) a compound of the general formula (VII):

$$R^{12}COCH_2A \qquad (VII)$$

in which $R^{12}$ and A have the meanings given above, and subsequently reducing the resulting compound of the general formula (VI′):

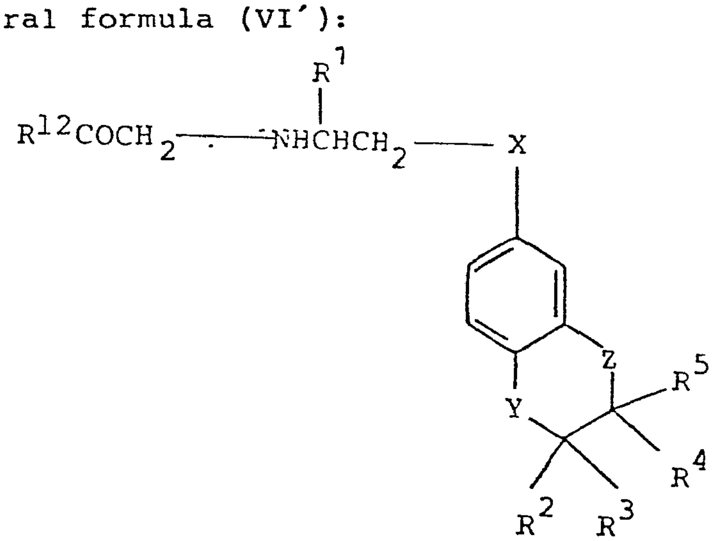

(VI′)

in which $R^1$, $R^2$, $R^3$, $R^4$, $R^5$, X and Y and Z are as defined in relation to formula (I) and $R^{12}$ is as defined in relation to formula (III), or a salt thereof; and, if desired, converting a resulting compound of the general formula (I) into another compound of the general formula (I) or into a pharmaceutically acceptable salt thereof.

A leaving group represented by A is any group that will, under the reaction conditions, cleave from the starting material thus promoting reaction at a specified site. Suitable examples of such groups are halogen atoms, mesyloxy groups and tosyloxy groups. Preferably in compounds (C) and (IV) A represents a tosyloxy group, or a bromine atom, and in compounds (D) and (VII) A represents a bromine atom.

Compounds of formulae (B), (C), (D), (F), (G), (J), (II), (III), (IV), (V), (VI), (VII) and (VI′) are either known compounds or can be prepared from known compounds by known processes or processes analogous to known processes.

For example, preparation of such materials may be analogous to the general methods of preparation described in European Patent Specification No. 0023385, where appropriate.

The reaction between compounds of the general formulae (II) and (V); or (E) and (J), is advantageously carried out in benzene, at reflux, in a Dean and Stark apparatus. The reaction between the compounds of the general formulae (II) and (VII); or (B) and (D), is

advantageously carried out in butanone or acetonitrile at reflux, if desired in the presence of a base. Subsequent reduction to obtain a compound of the general formula (A) or (I), may be carried out, for example with sodium borohydride, in situ or subsequent to isolation of any intermediate compound for example (VI) or (VI′). Preferably the subsequent reduction is carried out in situ.

The invention also provides a process for the preparation of a compound of the general formula (I) or a pharmaceutically acceptable salt thereof, which comprises reacting a compound of the general formula (VIII):

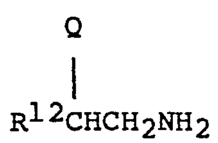

(VIII)

in which $R^{12}$ is as defined in relation to formula (III), and Q is as defined in relation to formula (I), or a salt or reactive derivative thereof, with

(i) a compound of the general formula (IX):

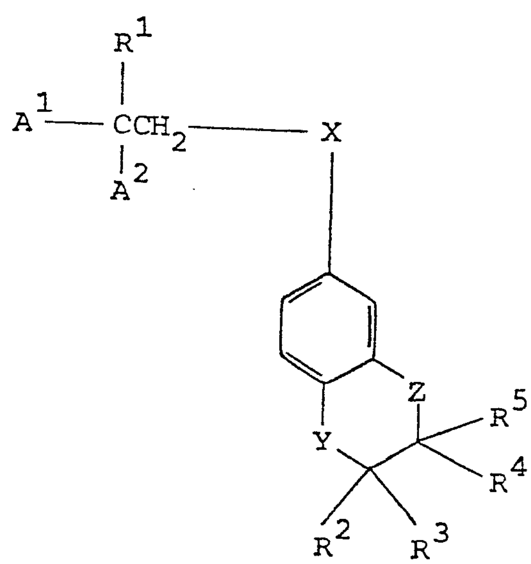

(IX)

in which $R^1$, $R^2$, $R^3$, $R^4$, $R^5$, X and Y and Z are as defined in relation to formula (I), $A^1$ represents a leaving group and $A^2$ represents a hydrogen atom, or

(ii) a compound of the general formula (IX) in which $R^1$, $R^2$, $R^3$, $R^4$, $R^5$, X and Y and Z have the meanings given above and $A^1$ and $A^2$ together represent an oxo group, and subsequently treating the intermediate with a reducing agent;
and, if desired, converting a resulting compound of the general formula (I) into another compound of the general formula (I) or into a pharmaceutically acceptable salt thereof.

Reaction of compounds of the general formula (VIII) and (IX) is advantageously carried out in the case where $A^1$ represents a leaving group, for example one of those mentioned above, more especially a tosyloxy group or a bromine atom, in dimethyl sulphoxide, for example at a temperature in the range of from 30 to 80°C, e.g. substantially 50°C and advantageously for a period of time of 1 to 4 days, e.g. about 3 days.

In the case where $A^1$ and $A^2$ together represent an oxo group, the reaction may be performed in several ways. For example, water may be removed azeotropically with benzene in a Dean and Stark apparatus and subsequent reduction carried out with sodium cyanoborohydride in a lower alkanol, preferably methanol, or with sodium borohydride in a lower alkanol, or by reductive catalysis using hydrogen and a palladium or platinum catalyst. Preferably the reduction is carried out using either sodium cyanoborohydride or sodium borohydride.

Method (ii) is a preferred method of preparation of compounds of the general formula (I).

Starting materials of the general formula (VIII) and (IX) are either known compounds or can be prepared from known compounds by known processes or processes analogous to known processes.

For example, the starting material of the general formula (X):

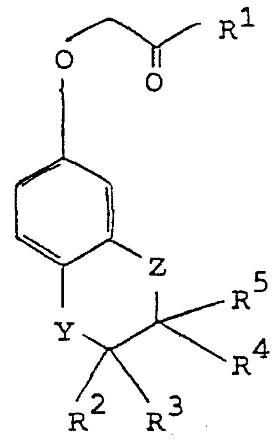

(X)

in which $R^1$, $R^2$, $R^3$, $R^4$, $R^5$, Y and Z are as defined in relation to formula (I) may be prepared, for example, by alkylating 6- or 7-hydroxybenzoxazinone with a suitable alkylating agent. This reaction is advantageously carried out in acetone using chloroacetone in the presence of an alkali metal carbonate, preferably potassium carbonate. The preparation of the reaction material

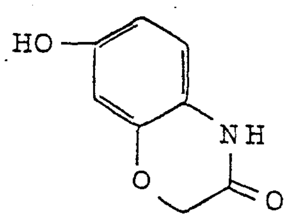

may be carried out, for example, as described in J.Chem. Soc., 739,1955. The preparation of the starting material

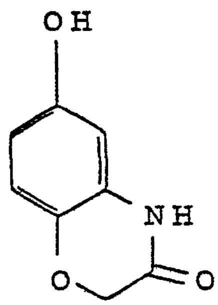

may be carried out, for example, as described in Swiss Patent Specification No. 308, 193 or C.A. 51, 7424F.

A compound of the general formula (A) or (I) may, if desired, be converted into another compound of the general formula (A) or (I) respectively by a method known *per se*. For example, an alcohol may be converted into an ester thereof.

Where necessary or desired, a free hydroxy, or amino group may be protected by protecting groups customary in the art during preparation of the compounds of the general formula (A) or (I). The protecting groups, may, if desired, be removed by the usual known methods subsequent to the preparation.

The present invention extends to novel intermediates and starting materials and thus further provides a compound of the general formula (E) or a compound of the general formula (II), or a compound of the general formula (G), or a compound of the general formula (VI) or (VI′) or a compound of the general formula (B) when $Y^1$ represents $X^2$, or a compound of the general formula (IX), or, where possible, a salt or reactive derivative thereof.

Compounds of the general formulae (II), (VI), (VI´), (IX); and (X) are useful starting materials or intermediates in the preparation of compounds of the general formula (I).

The pharmaceutically acceptable salts of compounds of the general formula (A) or (I) may be produced, for example, by treating a compound of general formula (I) with the appropriate acid.

Compounds of the general formula (A) or (I) and salts thereof, produced by the above processes, may be recovered by conventional methods.

Compounds of the general formula (A) or (I) may be separated into diastereoisomeric pairs of enantiomers by, for example, fractional crystallisation from a suitable solvent, for example methanol or ethyl acetate or a mixture thereof. The pair of enantiomers thus obtained may be separated into individual stereoisomers by conventional means, for example by the use of an optically active acid as a resolving agent.

Suitable optically active acids which may be used as resolving agents are described in ''Topics in Stereochemistry'', Vol. 6, Wiley Interscience, 1971, Allinger, N.L. and Eliel, W.L. Eds.

Alternatively, any enantiomer of a compound of the general formula (A) or (I) may be obtained by stereospecific synthesis using optically pure starting materials of known configuration.

For example, one enantiomer of compound (II) (when $R^1$ represents methyl) may be reacted with one enantiomer of a compound of the general formula (III) or one enantiomer of compound (VIII) may be reacted with one enantiomer of a compound of the general formula (IX) (when $A^1$ and $A^2$ do not represent oxo, and $R^1$ represents methyl), to produce a single enantiomer of the general formula (I).

Analogous processes to the abovementioned processes for the preparation of the compounds of formula (A) or formula (I), or pharmaceutically acceptable salts thereof, may be used to prepare any of the compounds of the present invention.

As previously indicated, the compounds of the present invention (hereinafter called ''the compound'') have valuable pharmacological properties.

Suitably 'the compound' is a compound of formula (A).

Favourably 'the compound' is a compound of formula (I).

The present invention therefore provides ''the compound'', or a pharmaceutically acceptable salt thereof, for use as an active therapeutic compound

The present invention further provides ''the compound'' or a pharmaceutically acceptable salt thereof, for use in the treatment of obesity in the human or non-human animal body.

The present invention further provides ''the compound'', or a pharmaceutically acceptable salt thereof, for use in the treatment of hyperglycaemia in the human or non-human animal body.

In a further aspect the present invention also provides ''the compound'', or a pharmaceutically acceptable salt thereof, for use in the treatment of cardiac disease or congestive heart failure in the human or non-human animal body.

'The compound 'or a pharmaceutically acceptable salt thereof may be administered per se or, preferably, as a pharmaceutical composition.

Accordingly, the present invention also provides a pharmaceutical composition comprising ''the compound'' or a pharmaceutically acceptable salt thereof, in admixture or conjunction with a pharmaceutically acceptable carrier therefore.

As used herein the terms ''pharmaceutical composition'' and ''pharmaceutically suitable'' embrace compositions and ingredients for both human and veterinary use.

The composition may, if desired, be in the form of a pack accompanied by written or printed instructions for use.

Usually the pharmaceutical composition of the present invention will be adapted for oral administration, although compositions for administration by other routes, such as by injection, are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or other conventional adjuvant.

Typical carriers include, for example, microcrystalline cellulose, starch, sodium starch glycollate, polyvinylpyrrolidone, polyvinylpolypyrrolidone, magnesium stearate, sodium lauryl sulphate or sucrose.

Most suitably the composition will be formulated in unit dose form. Such unit dose will normally contain an amount of the active ingredient in the range of from 0.1 to 500 mg, preferably 0.1 to 250 mg, and more especially 0.1 to 100 mg.

The present invention further provides a method for treating hyperglycaemia in a human or non-human animal which comprises administering an effective, non-toxic, amount of 'the compound' or a pharmaceutically acceptable salt thereof to a hyperglycaemic human or non-human animal.

The present invention further provides a method for treating obesity in a human or non-human animal, which comprises administering an effective, non-toxic, amount of ''the compound'' or a pharmaceutically acceptable salt thereof to an obese human or non-human animal.

Preferred compounds for use in treating hyperglycaemia and/or obesity are those compounds of the general formula (I) in which R represents an unsubstituted phenethanol radical or an $R^7$, $R^8$, $R^9$-substituted

phenethanol radical, especially those in which R of the general formula (I) represents a 2-hydroxy-2-[m-chlorophenyl]-ethyl radical.

The present invention further provides a method of treating cardiac disease or congestive heart failure in a human or non-human animal which comprises administering to the human or non-human animal an effective, non-toxic, amount of ''the compound'' or a pharmaceutically acceptable salt thereof.

Preferred compounds for use as positive inotropic agents are those compounds of general formula (I) in which R represents a 4-hydroxyphenoxypropan-2-ol radical or a 2-hydroxy-2-[4-hydroxyphenyl]-ethyl radical.

Conveniently, the active ingredient may be administered as a pharmaceutical composition as hereinbefore defined, and this forms a particular aspect of the present invention.

In treating humans in accordance with the present invention a compound of the general formula (I) or a pharmaceutically acceptable salt thereof may be taken in doses, such as those described above, one to six times a day in a manner such that the total daily dose for a 70 kg adult will generally be in the range of from about 0.1 to 1000 mg, and more usually about 1 to 500 mg. The total daily dosage range for humans will generally be in the range of $1.40 \times 10^{-3}$ mg/kg to 14.5 mg/kg, more usually $1.40 \times 10^{-2}$ mg/kg to 7.1 mg/kg.

In treating non-human animals, especially dogs, in accordance with the present invention, the active ingredient may be administered by mouth, usually once or twice a day and in an amount in the range of from about 0.025 mg/kg to 25 mg/kg, for example 0.1 mg/kg to 20 mg/kg.

In a further aspect the present invention also provides ''the compound'' or a pharmaceutically acceptable salt thereof for use in the manufacture of a medicament for the treatment of obesity and/or hyperglycaemia and/or cardiac disease or heart failure:

In a further aspect the present invention also provides a method for improving the weight gain and/or improving the feed utilisation efficiency and/or increasing lean body mass of livestock, which method comprises the administration to livestock of an effective non-toxic amount of 'the compound' or a veterinarily acceptable salt thereof.

Whilst 'the compound' and the veterinarily acceptable salts thereof may be administered to any livestock in the abovementioned method, they are particularly suitable for improving the weight gain and/or feed utilisation efficiency and/or lean body mass in poultry (especially turkeys and chickens), cattle, pigs and sheep.

In the preceding method ''the compound'' and veterinarily acceptable salts thereof will normally be administered orally although non-oral modes of administration, for example injection or implantation, are also envisaged. Suitably the compounds are administered in the feed-stuff or drinking water

provided for the livestock. Conveniently these are administered in the feed-stuff at from $10^{-3}$ppm - 500ppm of total daily feed intake, more usually 0.01ppm to 250ppm, suitably less than 100ppm.

The particular formulations used will of course depend upon the mode of administration but will be those used conventionally in the mode of administration chosen.

For administration in feed-stuff the drugs are conveniently formulated as a premix in association with a suitable carrier.

Accordingly, the present invention also provides a veterinarily acceptable premix formulation comprising 'the compound' or a veterinarily acceptable salt thereof in association with a veterinarily acceptable carrier.

Suitable carriers are inert conventional agents such as powdered starch. Other conventional feed-stuff premix carriers may also be employed.

No toxicological effects are indicated when ''the compound'' or a pharmaceutically acceptable salt thereof is administered in any of the above mentioned dosage ranges.

The following Examples illustrate the invention but do not limit it in any way.

Example 1

7-[2-[2-(4-Hydroxyphenyl)-2-hydroxyethylamino]-2-methylethoxy]-2H-1,4-benzoxazin-3-(4H)-one

Sodium cyanoborohydride (0.4 g) was added to a mixture of 2-[4-benzyloxyphenyl]-2-hydroxyethanolamine (1.0 g) and 7-(acetonyloxy)-2H-1,4-benzoxazinon-3-(4H)-one (0.9 g), in methanol at ambient temperature and the solution was allowed to stand 18h. The solvent was evaporated, the residue partitioned between ethyl acetate and water and the organic extracts dried ($MgSO_4$). Evaporation of the solvent gave an oil which was chromatographed on kieselgel. Elution with methanol/chloroform (2:98) gave 0.6 g of the corresponding 4-benzyloxy compound. This was dissolved in glacial acetic acid, 10% Pd/C added and the mixture hydrogenated at ambient temperature and pressure. The catalyst was filtered off and the solvent evaporated to give a residue which was partitioned between ethyl actate and sodium bicarbonate. The organic extracts were combined, washed with water and brine and dried ($MgSO_4$). Evaporation of the solvent gave 7-[2-[2-(4-hydroxyphenyl)-2-hydroxyethylamino]-2-methylethoxy]-2H-1,4-benzoxazin-3-(4H)-one as a 1:1 mixture of diastereoisomers, 0.3 g, m.p. 182-196°C.

$^1H$ nmr ($d_6$-DMSO), ppm

1.0 (3.0, 2 doublets), 2.5-2.7 (2H,m), 2.9 (1H,q), 2.6-3.5 (2H, broad, disappears with $D_2O$), 3.7 (2H, broad d), 4.5 (1H,m), 4.5 (2H,s), 6.4-6.85 (5H,m), 7.15 (2H,d), 9.3 (1H,broad), disappears with $D_2O$), 10.5 (1H, broad, disappears with $D_2O$).

Example 2

7-[2-[2-(3-Chlorophenyl)-2-hydroxyethylamino]-2-methylethoxy]-2H-1,4-benzoxazin-3-(4H)-one

7-[2-[2-(3-Chlorophenyl)-2-hydroxyethylamino]-2-methylethoxy]-2H-1,4-benzoxazin-3-(4H)-one was prepared as a 52:48 mixture of diastereoisomers, m.p. 134-140°C, from 2-[3-chlorophenyl)-2-hydroxyethanamine (0.39 g) and 7-(acetonyloxy)-2H-1,4-benzoxazin-3-(4H)-one (0.5 g), in an identical manner to the compound described in Example 1.

$^1$H nmr ($d_6$-DMSO), ppm

1.0 (3H, 2 doublets), 2.6-2.8 (2H,m), 2.95 (1H,q), 3.2 (1H, broad, disappears with $D_2O$), 2.75 (2H,d), 4.5 (2H,s), 4.6 (1H,m), 5.4 (1H, broad, disappears with $D_2O$), 6.45 (1H, dd), 6.52 (1H,d), 6.8 (1H,d), 7.2-7.5 (4H,m), 10.5 (1H, broad, disappears with $D_2O$).

Example 3

7-[2-[3-(4-Hydroxyphenoxy)-2-hydroxypropylamino]-2-methylethoxy]-2H-1,4-benzoxazin-3-(4H)-one

7-[2-[3-(4-Hydroxyphenoxy)-2-hydroxypropylamino]-2-methylethoxy]-2H-1,4-benzoxazin-3-(4H)-one was prepared as a 1:1 mixture of diastereoisomers, m.p. 150-166°C (isopropanol-ether), from 3-(4-benzyloxyphenoxy)-2-hydroxypropanamine (0.617 g) and 7-(acetonyloxy)-2H-1,4-benzoxazin-3-(4H)-one (0.5 g), in an identical manner to the compound described in Example 1. The hydrogenation was carried out using ethyl acetate/methanol as solvent and the product was chromatographed on kieselgel using methanol/ammonia/chloroform (10:0.5:89.5) as eluent.

$^1H$ nmr ($d_6$-DMSO), ppm

1.0 (3H,d), 2.5-2.7 (2H,m), 2.85 (1H,q), 3.3 (2H, broad, disappears with $D_2O$), 3.5-4.0 (5H,m), 4.5 (2H,s), 6.4-6.9 (7H,m), 8.85 (1h, broad, disappears with $D_2O$), 10.5 (1H, broad, disappears with $D_2O$).

Example 4

6-[2-[3-(4-Hydroxyphenoxy)-2-hydroxypropylamino]-2-methylethoxy]-2H-1,4-benzoxazin-3-(4H)-one

6-[2-[3-(4-Hydroxyphenoxy)-2-hydroxypropylamino]-2-methylethoxy]-2H-1,4-benzoxazin-3-(4H)-one was prepared as a 1:1 mixture of diastereoisomers, m.p. 113-121°C (ethyl acetate-methanol), from 3-(4-benzyloxyphenoxy)-2-hydroxypropanamine (0.435 g) and 6-(acetonyloxy)-2H-1,4-benzoxazin-3-(4H)-one (0.35 g), in an identical manner to the compound described in Example 3.

$^1H$ nmr ($d_6$-DMSO), ppm

1.0 (3H,d) 2.66 (2H,m) 2.9 (1H,q), 3.3 (1H, broad, disappears with $D_2O$), 3.5-3.9 (5H,m) 4.45 (2H,s), 4.9 (1H, broad, disappears with $D_2O$), 6.3-7.9 (7H,m), 8.8 (1H, broad, disappears with $D_2O$), 10.5 (1H, broad, disappears with $D_2O$).

Example 5

6-[2-[3-(2-Hydroxyphenoxy)-2-hydroxypropylamino]-2-methylethoxy]-2H-1,4-benzoxazin-3-(4H)-one hemifumarate

A mixture of 3-(2-benzyloxyphenoxy)-2-hydroxy-propanamine (0.62 g) and 6-(acetonyloxy)-2H-1,4-benzoxazin-3-(4H)-one (0.5 g), was heated under reflux for 2h in benzene using a Dean and Stark apparatus. The solvent was evaporated, the residue dissolved in methanol and sodium borohydride (0.25 g) added portionwise. After 0.25h the solvent was removed, the residue partitioned between ethyl acetate and water and the organic extracts dried ($MgSO_4$). The solvent was evaporated and the residue chromatographed on kieselgel. Elution with methanol/chloroform (2:98) gave 0.5 g of the corresponding 2-benzyloxy compound. This was dissolved in ethyl acetate, 10% Pd/C (0.1 g) added and the mixture hydrogenated at ambient temperature and pressure. The catalyst was filtered off, the solvent evaporated and the residue chromatographed on kieselgel. Elution with methanol/chloroform (8:92) gave an oil (0.15 g) which was treated with fumaric acid (0.024 g) in methanol to give 6-[2-[3-(2-hydroxyphenoxy)-2-hydroxypropylamino]-2-methylethoxy]-2H-1,4-benzoxazin-3-(4H)-one hemifumarate

as a 55:45 mixture of diastereoisomers, m.p. 178-185°C (methanol-ethyl acetate).

$^1H$ nmr ($d_6$-DMSO), ppm

1.1-1.2 (3H, 2 doublets), 2.7-3.0 (2H,m), 3.1 (1H,q), 3.3-3.7 (2H, broad, disappears with $D_2O$), 3.7-4.1 (5H,m), 4.48 (2H,s), 6.5 (2H,m), 6.54 (1H,s), 6.68-6.91 (5H,m), 10.6 (1H, disappears with $D_2O$).

Example 6

6-[2-[2-(3-Chlorophenyl)-2-hydroxyethylamino]-2-methylethoxy]-2H-1,4-benzoxazin-3-(4H)-one

A mixture of 2-(3-chlorophenyl)-2-hydroxyethanamine (0.4 g) and 6-(acetonyloxy)-2H-1,4-benzoxazin-3-(4H)-one (0.51 g), was heated under reflux for 4h in benzene using a Dean and Stark apparatus. The solvent was evaporated, the residue dissolved in methanol, sodium cyanoborohydride added and the solution allowed to stand at ambient temperature overnight. The solvent was evaporated, the residue partitioned between ethyl acetate and water and the organic extracts dried ($MgSO_4$) and evaporated. The residue was chromatographed on kieselgel. Elution with methanol/chloroform (2:98) gave 6-[2-[2-(3-chlorophenyl)--2-hydroxyethylamino]-2-methylethoxy]-2H-1,4-benzoxazin-3-(4H)-one as a 56:44 mixture of diastereoisomers, m.p. 120-125°C (ethyl acetate-cyclohexane).

$^1H$ nmr ($d_6$-DMSO), ppm

1.05 (3H, 2 doublets), 2.0 (1H, broad, disappears with

$D_2O$), 2.6-2.8 (2H,m) 2.95 (1H,q), 3.8 (2H,m), 4.5 (2H,s), 4.65 (1H,t), 5.48 (1H, broad, disappears with $D_2O$), 6.4-6.6 (2H,m), 6.87 (1H,d), 7.2-7.5 (4H,m), 10.6 (1H, broad, disappears with $D_2O$).

Example 7

6-[2-[2-(3,4-Dichlorophenyl)-2-hydroxyethylamino]-2-methylethoxy]-2H-1,4-benzoxazin-3-(4H)-one

6-[2-[2-(3,4-Dichlorophenyl)-2-hydroxyethylamino]-2-methylethoxy]-2H-1,4-benzoxazin-3-(4H)-one was prepared as a mixture of diastereoisomers, m.p. 145-155°C (acetone), from 2-(3,4-dichlorophenyl)-2-hydroxyethanamine (0.47 g) and 6-(acetonyloxy)-2H-1,4-benzoxazin-3-(4H)-one (0.5 g) in an identical manner to the compound described in Example 6.

$^1H$ nmr ($d_6$-DMSO), ppm

1.05 (3H,d), 1.9 (1H, broad, disappears with $D_2O$), 2.5-2.80 (2H,m), 2.8-3.1 (1H,m), 3.72 (2H,d), 4.48 (2H,s), 4.6 (1H,m), 5.5 (1H, broad, disappears with $D_2O$), 6.35-6.6 (2H,m), 6.85 (1H,d), 7.35 (1H,dd), 7.55 (2H,m), 10.6 (1H, broad, disappears with $D_2O$).

EXAMPLE 8

6-[2-[2-(3-Chlorophenyl)-2-hydroxyethylamino]-2-methylethyl]-2H-1,4-benzoxazin-3-(4H)-one

6-[2-[2-(3-Chlorophenyl)-2-hydroxyethylamino]-2-methylethyl]-2H-1,4-benzoxazin-3-(4H)-one was prepared as a 14:86 mixture of diastereoisomers, m.p. 130-142°C (cyclohexane/ethyl acetate), from 2-(3-chlorophenyl)-2-hydroxyethanamine (0.7 g) and 6-acetonyl-2H-1,4-benzoxazin-3-(4H)-one (0.82 g) in an identical manner to the compound described in Example 6. Crystallisation of the mother liquors from acetone afforded 6-[2-[2-(3-chlorophenyl)-2-hydroxyethylamino]-2-methylethyl]-2H-1,4-benzoxazin-3-(4H)-one as a 82:18 mixture of diastereoisomers, m.p. 145-153°.

$^1$H nmr ($d_6$-DMSO + $D_2O$), ppm

0.95 (3H,d); 2.3-2.9 (5H,m); 4.5 (2H,s); 4.55 (1H,m); 6.6-6.9 (3H,m); 7.2-7.35 (4H,m).

EXAMPLE 9

6-[2-[3-(2-Hydroxyphenoxy)-2-hydroxypropylamino]-2-methylethyl]-2H-1,4-benzoxazin-3-(4H)-one hemifumarate

6-[2-[3-[2-Hydroxyphenoxy)-2-hydroxypropylamino]-2-methylethyl]-2H-1,4-benzoxazin-3-(4H)-one hemifumarate was prepared as a 49:51 mixture of diastereoisomers, m.p. 182-184$^{O}$ (methanol), from 3-(2-benzyloxyphenoxy)-2-hydroxypropanamine (1.3 g) and 6-acetonyl-2H-1,4-benzoxazin-3-(4H)-one (0.96 g) in an identical manner to the compound described in Example 5.

$^{1}H$ nmr ($d_6$-DMSO), ppm

1.03 (3H,d); 2.6-3.3 (5H,m); 3.75-4.2 (3H,m); 4.52 (2H,s); 4.7-5.9 (4H, broad, disappears with $D_2O$); 6.5 (1H,s); 6.6-7.0 (7H,m); 10.3-11.0 (1H, broad, disappears with $D_2O$).

EXAMPLE 10

6-[2-[2-Phenyl-2-hydroxyethylamino]-2-methylethyl]-2H-1,4-benzoxazin-3-(4H)-one

6-[2-[2-Phenyl-2-hydroxyethylamino]-2-methylethyl]-2H-1,4-benzoxazin-3-(4H)-one was prepared as a 52:48 mixture of diastereoisomers, m.p. 144-164$^{o}$ (acetone), from 2-hydroxy-2-phenylethylamine (1.0 g) and 6-acetonyl-2H,-1,4-benzoxazin-3-(4H)-one (1.50 g) in an identical manner to the compound described in Example 6.

$^{1}H$ nmr ($d_6$-DMSO), ppm

0.95 (3H,d); 2.3-2.9 (5H,m); 3.0-3.5 (1H, broad, disappears with $D_2O$); 4.52 (2H,s); 4.5-4.65 (1H,m); 4.9-5.5 (1H, broad, disappears with $D_2O$); 6.6-6.95 (3H,m); 7.3 (5H, bs); 10.0-11.5 (1H, broad, disappears with $D_2O$).

EXAMPLE 11

6-[2-[3-Phenoxy-2-hydroxypropylamino]-2-methylethyl]-2H-1,4-benzoxazin-3-(4H)-one

6-[2-[3-Phenoxy-2-hydroxypropylamino]-2-methylethyl]-2H-1,4-benzoxazin-3-(4H)-one was prepared as a 45:55 mixture of diastereoisomers, m.p. 128-136$^{o}$ (acetone), from 2-hydroxy-3-phenoxypropylamine (1.0 g) and 6-acetonyl-2H-1,4-benzoxazin-3-(4H)-one (1.23 g) in an identical manner to the compound described in Example 6.

$^{1}$H nmr ($d_6$-DMSO), ppm

0.95 (3H,d); 2.3-3.0 (5H,m); 3.3 (1H, broad, disappears with $D_2O$); 3.7-4.0 (3H,m); 4.55 (2H,s); 4.95 (1H, broad, disappears with $D_2O$); 6.65-7.5 (8H,m); 9.5-11.5 (1H, broad disappears with $D_2O$).

EXAMPLE 12

6-[2-[2-(3-Chlorophenyl)-2-hydroxyethylamino]-2-methylethoxy]-4-methyl-2H-1,4-benzoxazin-3-(4H)-one hemifumarate

6-[2-[2-(3-Chlorophenyl)-2-hydroxyethylamino]-2-methylethoxy]-4-methyl-2H-1,4-benzoxazin-3-(4H)-one hemifumarate was prepared as a 48:52 mixture of diastereoisomers, m.p. 160-182$^{O}$ (methanol), from 2-(3-chlorophenyl)-2-hydroxyethylamine (1.67 g) and 6-acetonyloxy-4-methyl-2H-1,4-benzoxazin-3-(4H)-one in an identical manner to the compound described in Example 6.

$^{1}$H nmr ($d_6$-DMSO), ppm

1.15 (3H,d); 2.6-3.2 (3H,m); 3.25 (3H,s); 3.94 (2H,d); 4.55 (2H,s); 4.8 (1H,m); 6.15 (3H, broad, disappears with $D_2O$); 6.55 (1H,s); 6.55-7.0 (3H,m); 7.3-7.55 (4H,m).

EXAMPLE 13

6-[2-[2-(3-Chlorophenyl)-2-hydroxyethylamino]-2-methylethyl]-4-methyl-2H-1,4-benzoxazin-3-(4H)-one hemifumarate

6-[2-[2-(3-Chlorophenyl)-2-hydroxyethylamino]-2-methylethyl]-4-methyl-2H-1,4-benzoxazin-3-(4H)-one hemifumarate was prepared as a 64:36 mixture of diastereoisomers, m.p. 176-182$^{O}$ (methanol - ethyl acetate), from 2-(3-chlorophenyl)-2-hydroxyethylamine (0.90 g) and 6-acetonyl-4-methyl-2H-1,4-benzoxazin-3-(4H)-one (0.49 g) in an identical manner to the compound described in Example 6.

$^{1}$H nmr ($d_6$-DMSO), ppm

1.05 (3H,d); 2.5-3.3 (5H,m); 3.28 (3H,s); 4.6 (2H,s); 4.8 (1H,m); 6.55 (1H,s); 6.6 (3H, broad, disappears with $D_2O$); 6.8-7.1 (3H,m); 7.3-7.6 (4H,m).

EXAMPLE 14

6-[2-[3-(4-Hydroxyphenoxy)-2-hydroxypropylamino]-2-methylethyl]-2H-1,4-benzoxazin-3-(4H)-one

6-[2-[3-(4-Hydroxyphenoxy)-2-hydroxypropylamino]-2-methylethyl]-2H-1,4-benzoxazin-3-(4H)-one was prepared as a 40:60 mixture of diastereoisomers, m.p. 143-147$^{o}$ (acetone), from 3-(4-benzyloxyphenoxy)-2-hydroxypropanamine (1.57 g) and 6-acetonyl-2H-1,4-benzoxazin-3-(4H)-one (1.18 g) in an identical manner to the compound described in Example 1.

$^{1}H$ nmr ($d_6$-DMSO + $D_2O$), ppm

0.95 (3H,d); 2.4-2.9 (5H,m); 3.88 (bs, 3H), 4.5 (2H,s); 6.6-7.0 (7H,m).

EXAMPLE 15

6-[2-[2-(4-Hydroxyphenyl)-2-hydroxyethylamino]-2-methylethoxy]-2H-1,4-benzoxazin-3-(4H)-one

6-[2-[2-(4-Hydroxyphenyl)-2-hydroxyethylamino]-2-methylethoxy]-2H-1,4-benzoxazin-3-(4H)-one was prepared as a 31:69 mixture of diastereoisomers, m.p. 167-175$^{O}$ (acetone) from 2-(4-benzyloxyphenyl)-2-hydroxyethylamine (1.05 g) and 6-acetonyl-2H-1,4-benzoxazin-3-(4H)-one (1.08 g) in an identical manner to the compound described in Example 5.

$^{1}$H nmr ($d_6$-DMSO), ppm

1.05 (3H, 2 doublets); 2.6 (2H,m); 2.8 (1H, broad, disappears with $D_2O$); 2.95 (1H,m); 3.7 (2H,d); 4.5 (2H,s); 4.5 (1H,m); 5.1 (1H, broad, disappears with $D_2O$); 6.4-7.3 (7H,m); 9.2 (1H, broad, disappears with $D_2O$); 10.6 (1H, broad, disappears with $D_2O$).

EXAMPLE 16

6-[2-[3-(2-Allyloxyphenoxy)-2-hydroxypropylamino]-2-methyl-ethoxy]-2H-1,4-benzoxazin-3-(4H)-one

6-[2-[3-(2-Allyloxyphenoxy)-2-hydroxypropylamino]-2-methyl-ethoxy]-2H-1,4-benzoxazin-3-(4H)-one was prepared as a 39:61 mixture of diastereoisomers, m.p. 136-141$^{o}$ (methanol) from 3-(2-allyloxyphenoxy)-2-hydroxypropanamine (1.0 g) and 6-acetonyloxy-2H-1,4-benzoxazin-3-(4H)-one (1.0 g) in an identical manner to the compound described in Example 6.

$^{1}$H nmr ($d_6$-DMSO), ppm

1.05 (3H,d); 2.5-3.1 (3H,m); 3.3 (1H, broad, disappears with $D_2O$); 3.6-4.0 (5H,m); 4.5 (2H,s); 4.6 (2H,m); 4.95 (1H, broad disappears with $D_2O$); 5.1-5.5 (2H,m); 5.8-6.3 (1H,m); 6.35-6.65 (2H,m); 6.75-7.1 (5H,m).

EXAMPLE 17

6-[2-[3-(2-Methoxyphenoxy)-2-hydroxypropylamino]-2-methyl-ethoxy]-2H-1,4-benzoxazin-3-(4H)-one

6-[2-[3-(2-Methoxyphenoxy)-2-hydroxypropylamino]-2-methyl-ethoxy]-2H-1,4-benzoxazin-3-(4H)-one was prepared as a 67:33 mixture of diastereoisomers, m.p. 143-160$^{o}$ (methanol) from 3-(2-methoxyphenoxy)-2-hydroxypropanamine (1.0 g) and 6-acetonyloxy-2H-1,4-benzoxazin-3-(4H)-one (1.12 g) in an identical manner to the compound described in Example 6.

$^{1}H$ nmr ($d_6$-DMSO), ppm

1.05 (3H,d); 2.5-3.1 (3H,m); 3.3 (1H, broad, disappears with $D_2O$); 3.75 (3H,s); 3.7-4.0 (5H,m); 4.5 (2H,s); 4.95 (1H, broad, disappears with $D_2O$); 6.35-6.6 (2H,m); 6.7-7.1 (5H, m); 10.6 (1H, broad, disappears with $D_2O$).

EXAMPLE 18

6-[2-(2-Hydroxy-3-phenoxypropylamino)-2-methylethoxy]-2H-1,4-benzoxazin-3-(4H)-one

6-[2-(2-Hydroxy-3-phenoxypropylamino)-2-methylethoxy]-2H-1,4-benzoxazin-3-(4H)-one was prepared as a 46:54 mixture of diastereoisomers, m.p. 96-106$^{o}$ (acetone) from 2-hydroxy-3-phenoxypropanamine (1.0 g) and 6-acetonyloxy-2H-1,4-benzoxazin-3-(4H)-one (1.32 g) in an identical manner to the compound described in Example 6.

$^{1}H$ nmr ($d_6$-DMSO), ppm

1.05 (3H,d); 2.5-3.1 (3H,m); 3.3 (1H, broad, disappears with $D_2O$); 3.6-4.0 (5H,m); 4.5 (2H,s); 4.95 (1H, broad, disappears with $D_2O$); 6.35-6.6 (2H,m); 6.7-7.05 (4H,m); 7.1-7.35 (2H,m); 10.6 (1H, broad, disappears with $D_2O$).

EXAMPLE 19

6-[2-[2-Hydroxy-3-(indol-4-yloxy)propylamino]-2-methylethoxy]-2H-1,4-benzoxazin-3-(4H)-one

6-[2-[2-Hydroxy-3-(indol-4-yloxy)propylamino]-2-methylethoxy]-2H-1,4-benzoxazin-3-(4H)-one was prepared as a 56:44 mixture of diastereoisomers, m.p. 138-148$^{o}$ (acetone), from 2-hydroxy-3-(indol-4-yloxy)propanamine (0.92 g) and 6-acetonyloxy-2H-1,4-benzoxazin-3-(4H)-one (0.98 g) in an identical manner to the compound described in Example 6.

$^{1}H$ nmr ($d_6$-DMSO), ppm

1.1 (3H,d); 2.55-3.1 (3H,m); 3.3 (1H, broad, disappears with $D_2O$); 3.7-4.2 (5H,m); 4.5 (2H,s); 5.02 (1H,broad, disappears with $D_2O$); 6.35-6.7 (4H,m); 6.8-7.35 (4H,m)-10.6 (1H, broad, disappears with $D_2O$); 11.1 (1H, broad, disappears with $D_2O$).

EXAMPLE 20

6-[2-[2-(7-Ethylbenzofuran-2-yl)-2-hydroxyethylamino]-2-methylethoxy]-2H-1,4-benzoxazin-3-(4H)-one

6-[2-[2-(7-Ethylbenzofuran-2-yl)-2-hydroxyethylamino]-2-methylethoxy]-2H-1,4-benzoxazin-3-(4H)-one was prepared as a 54:46 mixture of diastereoisomers, m.p. 143-150$^{o}$ (acetone) from 2-(7-ethylbenzofuran-2-yl)-2-hydroxyethylamine (0.42 g) and 6-acetonyloxy-2H-1,4-benzoxazin-3-(4H)-one (o.45 g) in an identical manner to the compound described in Example 6.

$^{1}$H nmr ($d_6$-DMSO), ppm

1.05 (3H,d); 1.25 (3H,t); 2.8 (2H,q); 2.8-3.1 (3H,m); 3.3 (lH, broad, disappears with $D_2O$); 3.7 (2H,d); 4.5 (2H,s); 4.75 (lH,m); 5.6 (lH, broad, disappears with $D_2O$); 6.3-6.5 (2H,m); 6.65-6.9 (2H,m); 7.0-7.25 (2H,m); 7.3-7.5 (lH,m); 10.6 (lH, broad, disappears with $D_2O$).

EXAMPLE 21

6-[2-[3-(3,5-Dihydroxyphenoxy)-2-hydroxypropylamino]-2-methylethoxy]-2H-1,4-benzoxazin-3-(4H)-one hemifumarate

6-[2-[3-(3,5-Dihydroxyphenoxy)-2-hydroxypropylamino]-2-methylethoxy]-2H-1,4-benzoxazin-3-(4H)-one hemifumarate was prepared as a 47:53 mixture of diastereoisomers, m.p. 224-230° (ethanol-ethyl acetate), from 3-(3,5-dibenzyloxyphenoxy)-2-hydroxypropanamine (1.05 g) and 6-acetonyloxy-2H-1,4-benzoxazin-3-(4H)-one (0.61 g) in an identical manner to the compound described in Example 5.

$^1H$ nmr ($d_6$-DMSO), ppm

1.25 (3H,d); 2.6-3.3 (3H,m); 3.7-4.2 (5H,m); 4.5 (2H,s); 5.9 (3H,s); 6.4-6.7 (3H,m); 6.8-7.0 (1H,m).

EXAMPLE XI

7-(Acetonyloxy)-2H-1,4-benzoxazin-3-(4H)-one

Chloroacetone (0.96 ml) in acetone (15 ml) was added dropwise over 1h to a mixture of potassium carbonate (2.0 g) and 7-hydroxy-2H-1,4-benzoxazin-3-(4H)-one (2.0 g) (prepared according to J. Chem. Soc. 1955, 739) in acetone at reflux. The mixture was refluxed for 5h. The solvent was evaporated, the residue partitioned between water and ethyl acetate and the organic extracts dried ($MgSO_4$) and evaporated. The residue was chromatographed on kieselgel. Elution with chloroform gave the 4,7-dialkylated material (~ 0.2 g) followed by 7-(acetonyloxy)-2H-1,4-benzoxazin-3-(4H)-one (0.9 g).

$^1$H nmr ($d_6$-DMSO), ppm

2.2 (3H,s); 4.6 (2H,s); 4.78 (2H,s); 6.4-6.7 (2H,m); 6.9 (1H,d); 10.5 (1H,s).

EXAMPLE X2



6-(Acetonyloxy)-2H-1,4-benzoxazin-3-(4H)-one (Preparation 1)

6-(Acetonyloxy)-2H-1,4-benzoxazin-3-(4H)-one was prepared from 6-hydroxy-2H-1,4-benzoxazin-3-(4H)-one (Swiss Patent 308,193 (1955), C.A. 51, 7424F) in an identical manner to the compound described in Example 23.

$^1H$ nmr ($CDCl_3$ + $d_6$-DMSO), ppm

2.3 (3H,s); 4.58 (4H,s); 6.3-6.6 (2H,m); 6.72 (1H,d); 10.4 (1H, broad).

EXAMPLE X3

6-Acetonyloxy-2H-1,4-benzoxazin-3-(4H)-one
(Preparation 2)

A mixture of 5-acetonyloxy-2-ethoxycarbonylmethoxynitrobenzene (18 g) in ethyl acetate (150 ml) and glacial acetic acid (50 ml) and 10% Pd/C (1.8 g) was hydrogenated at ambient temperature and atmospheric pressure until hydrogen uptake ceased. The mixture was filtered and the filtrate evaporated to give a brown solid residue. This residue was chromatographed in kieselgel using chloroform as eluent to give 6-acetonyloxy-2H-1,4-benzoxazin-3-(4H)-one as a white solid, m.p. 162-165$^{o}$.

$^{1}H$ nmr ($d_6$-DMSO), ppm

2.15 (3H,s); 4.5 (2H,s); 4.7 (2H,s); 6.3-6.6 (2H,m); 6.8-6.95 (1H,m); 10.7 (1H, bs, replaceable by $D_2O$).

EXAMPLE X4

0171702

5-Acetonyloxy-2-ethoxycarbonylmethoxynitrobenzene

A mixture of 2-ethoxycarbonylmethoxy-5-hydroxynitrobenzene (16.5 g), potassium carbonate (9.45 g) and chloroacetone (7.5 ml) in methyl ethyl ketone (250 ml) was stirred and heated at reflux for 16 hours. The mixture was cooled to ambient temperature, filtered and the filtrate evaporated. The residue was chromatographed on kieselgel, using diethyl ether/60-80 petrol (60:40) as eluent, to yield 5-acetonyloxy-2-ethoxycarbonylmethoxynitrobenzene as a yellow solid, m.p. 72-75$^{o}$ (18.2 g).

$^{1}$H nmr ($CDCl_3$), ppm

1.28 (3H,t); 2.26 (3H,s); 4.25 (2H,q); 4.60 (2H,s); 4.74 (2H,s); 6.9-7.1 (2H,m); 7.35 (1H,d).

EXAMPLE X5

2-Ethoxycarbonylmethoxy-5-hydroxynitrobenzene

To a solution of 2-ethoxycarbonylmethoxy-5-tetrahydropyranyloxynitrobenzene (22.5 g) in ethanol (120 ml), at ambient temperature, was added 2M HCl (20 ml). After 1h the ethanol was removed in vacuo, the aqueous residue diluted with water (100 ml) and the combined aqueous phase extracted with diethyl ether. The ether extract was washed with water, dried ($MgSO_4$) and evaporated to give 2-ethoxycarbonylmethoxy-5-hydroxynitrobenzene as a yellow crystalline solid, m.p. 73-76.5°C (16.5 g).

$^1H$ nmr ($CDCl_3$ + $d_6$-DMSO), ppm

1.3 (3H,t); 4.25 (2H,q); 4.70 (2H,s); 6.9-7.1 (2H,m); 7.35 (1H,d); 9.3 (1H, broad, replaceable by $D_2O$).

EXAMPLE X6

2-Ethoxycarbonylmethoxy-5-tetrahydropyranyloxynitrobenzene

A mixture of 2-hydroxy-5-tetrahydropyranyloxynitrobenzene (21 g), potassium carbonate (12.2 g) and ethyl bromoacetate (9.75 ml) in methyl ethyl ketone (250 ml) was stirred and heated at reflux for 3 hours. The mixture was cooled to ambient temperature, filtered, and the filtrate concentrated under reduced pressure to give a yellow oil, which was chromatographed on kieselgel using 60-80 petrol/diethyl ether (80:20) as eluent. This gave 2-ethoxycarbonylmethoxy-5-tetrahydropyranyloxynitrobenzene as a yellow solid (22.5 g), m.p. 43-46°C.

$^1$H nmr ($CDCl_3$), ppm

1.25 (3H,t); 1.3-2.2 (6H,m); 3.3-4.0 (2H,m); 4.25 (2H,q); 4.70 (2H,s); 5.35 (1H,m); 7.0 (1H,d); 7.25 (1H,dd); 7.6 (1H,d).

EXAMPLE X7

2-Hydroxy-5-tetrahydropyranyloxynitrobenzene

To a stirred suspension of 2-nitroquinol (16.3 g) in dry dichloromethane (190 ml) at ambient temperature, was added dihydropyran (19.17 ml) and p-toluenesulphonic acid monohydrate (190 mg). After ten minutes, the resulting solution was diluted with diethyl ether (350 ml) and the organic solution washed with a solution made up of aqueous saturated sodium chloride (100 ml), aqueous saturated sodium bicarbonate (100 ml) and water (200 ml). The organic phase was separated, washed again with saturated sodium chloride solution, dried over magnesium sulphate and evaporated to give an oil. Chromatography on kieselgel using petroleum ether, b.p. 60-80/diethyl ether (80:20) as eluent gave 2-hydroxy-5-tetrahydropyranyloxynitrobenzene as an oil (21 g).

$^1H$ nmr ($CDCl_3$), ppm

1.3-2.1 (6H,m); 3.3-4.0 (2H,m); 5.3 (1H,m); 7.10 (1H,d); 7.30 (1H,dd); 7.80 (1H,d); 10.25 (1H,s, replaceable by $D_2O$).

EXAMPLE X8

6-Acetonyl-2H-1,4-benzoxazin-3-(4H)-one

A solution of 1-(4-ethoxycarbonylmethoxy-3-nitrophenyl)-2-nitroprop-1-ene (15 g) in glacial acetic acid (210 ml) was warmed, with mechanical stirring, to 90°C. Iron powder (42 g) and water (100 ml) were then added alternately in portions over 15 minutes (exothermic reaction). The resulting mixture was stirred a further hour at 90°C. After cooling to ambient temperature, the mixture was filtered through celite and the filter cake washed with dichloromethane. The combined filtrates were evaporated under reduced pressure, the residue partitioned between ethyl acetate and water and the biphasic solution filtered again through celite. The organic phase was separated, washed with aqueous 4N sodium carbonate (x 2), water, dried over magnesium sulphate and evaporated to give a yellow crystalline solid. Crystallisation from ethanol gave 6-acetonyl-2H-1,4-benzoxazin-3-(4H)-one (8 g), m.p. 160-164°C.

$^1$H nmr ($CDCl_3$), ppm

2.17 (3H,s); 3.60 (2H,s); 4.60 (2H,s); 6.65-7.05 (3H,m); 9.05-9.6 (1H, bs, exchanges with $D_2O$).

EXAMPLE X9

1-(4-Ethoxycarbonylmethoxy-3-nitrophenyl)-2-nitroprop-1-ene

To a solution of 1-(4-hydroxy-3-nitrophenyl)-2-nitroprop-1-ene (17.5 g) in acetone (250 ml), was added ethylbromoacetate (8.6 ml) and anhydrous potassium carbonate (10.8 g). The mixture was stirred and heated at reflux for 20 hours, cooled to ambient temperature, filtered and the filtrate evaporated. The residue was dissolved in ethyl acetate, washed with 2M sodium hydroxide solution, water, dried ($MgSO_4$) and evaporated to give a solid. The solid was crystallised from ethanol to give 1-(4-ethoxycarbonylmethoxy-3-nitrophenyl)-2-nitroprop-1-ene (15 g).

$^1H$ nmr ($CDCl_3$), ppm

1.32 (3H, t, J=7Hz); 2.46 (3H,s); 4.25 (2H,q, J=7Hz); 4.83 (2H,s); 7.18 (1H,d, J=8Hz); 7.64 (1H, dd, J=8Hz, J=2Hz); 7.95 (1H,s + 1H,d).

EXAMPLE X10



1-(4-Hydroxy-3-nitrophenyl)-2-nitroprop-1-ene

A solution of 4-hydroxy-3-nitrobenzaldehyde (25 g) and n-butylamine (16.2 ml) in benzene (200 ml) was heated under reflux for 2 hours with azeotropic removal of water using a Dean and Stark trap. The solution was cooled and evaporated under reduced pressure. The residue was dissolved in glacial acetic acid (100 ml) and nitroethane (11 ml) added. After stirring and heating at 100$^{o}$C for 1 hour, the solution was cooled to room temperature during which time crystals separated. The crystals were filtered, washed with ether and dried to give 1-(4-hydroxy-3-nitrophenyl)-2-nitroprop-1-ene (20 g).

$^{1}$H nmr ($d_6$-DMSO), ppm

2.4 (3H,s); 7.15 (1H,d, J=8Hz); 7.84 (1H,dd, J=8Hz, J=2Hz); 7.95-8.1 (1H,s + 1H,d); 9.9-11.2 (1H, bs, exchange with $D_2O$).

DEMONSTRATION OF EFFECTIVENESS OF COMPOUNDS

1. Effect on Energy Expenditure of Mice

The effect of the compounds on the energy expenditure of mice was demonstrated by means of the following procedure:

Female CD1 mice each weighing approximately 24 g, were given food and water ad lib before and during the experiment. The compounds were dissolved in water and these solutions were administered orally to each of 12 mice. A further 12 mice were dosed orally with water. The mice were placed in boxes through which air was drawn and the oxygen content of the air leaving the boxes was measured. The energy expenditure of the mice was calculated for 3 and 21 hours after dosing from the volume of air leaving the boxes and its oxygen content, following the principles described by J.B. de V. Weir, J. Physiol. (London) 109, 1-9, (1949). The results are expressed as a percentage of the rate of energy expenditure of the mice dosed with water.

| Compounds of Example No. | Dose mg/kg p.o. | Mean Energy Expenditure | |
|---|---|---|---|
| | | 0-3h | 0-21h |
| 1 | 17.9 | 165 | 104 |
| 2 | 18.9 | 164 | 119 |
| 3 | 19.9 | 99 | 100 |
| 4 | 19.4 | 142 | 115 |
| 5 | 22.3 | 123 | 112 |
| 6 | 18.8 | 141 | 115 |
| 7 | 20.6 | 120 | 105 |
| 8 (14:86) | 18.0 | 120 | 108 |
| 8 (82:18) | 18.0 | 145 | 111 |
| 9 | 21.5 | 119 | 99 |
| 10 | 16.3 | 104 | 106 |
| 11 | 18.0 | 133 | 111 |
| 12 | 22.4 | 116 | 103 |
| 13 | 21.6 | 129 | 109 |
| 18 | 18.6 | 165 | 109 |
| 19 | 20.6 | 104 | 106 |

2. Effect on Energy Expenditure of Rats

The effect of the compounds on the energy expenditure of rats was demonstrated by means of the following procedure:

Male Sprague-Dawley rats each weighing between 170-200 g were deprived of food for 16 hours before, and during the experiment. Water was provided ad lib at all times. The compounds were administered orally in water to 3 or 4 rats. A further 4 rats were dosed orally with water. The rats were placed in boxes through which air was drawn and the oxygen content of the air leaving the boxes was measured. The energy expenditure of the rats was calculated for 3 hours and for 21 hours after dosing from the volume of air leaving the boxes and its oxygen content, following the principles described by J.B. de V. Weir, J. Physiol. (London) 109, 1-9 (1949). The results are expressed as a percentage of the rate of energy expenditure of the rats dosed with water.

| Compounds of Example No. | Dose mg/kg p.o. | Mean Energy Expenditure 0-3h | 0-21h |
|---|---|---|---|
| 2 | 7.5 | 128 | 110 |
| 3 | 4.0 | 108 | 105 |
| 4 | 7.8 | 128 | 119 |
| 5 | 8.9 | 111 | 100 |
| 6 | 7.5 | 125 | 105 |
| 7 | 4.1 | 149 | 107 |
| 8 (14:86) | 3.6 | 125 | 105 |
| 8 (82:18) | 3.6 | 122 | 106 |
| 10 | 32.6 | 122 | 101 |
| 11 | 7.1 | 119 | 108 |
| 12 | 8.9 | 109 | 97 |
| 14 | 3.7 | 114 | 103 |
| 15 | 7.2 | 126 | 103 |
| 16 | 4.3 | 115 | 101 |
| 17 | 4.0 | 112 | 103 |
| 18 | 3.7 | 155 | 109 |
| 19 | 8.2 | 116 | 114 |
| 20 | 4.1 | 110 | 107 |
| 21 | 21.6 | 110 | 105 |

3. Hypoglycaemic Activity

Female CFLP mice, weighing approximately 25 g, were fasted for 24 hours prior to the study. The compounds under study were administered orally as an aqueous solution to each of 6 mice. 30 minutes later a blood sample (20 µl) was obtained from the tail for the analysis of blood glucose. Immediately after taking this blood sample, glucose (1 g/kg body weight) was administered subcutaneously to each mouse. 6 mice were given water as a control. Blood samples were then obtained from each mouse at 30 minute intervals for 120 minutes.

Compounds that produced a significant ($p<0.05$) reduction of blood glucose, compared with control mice given water, at any time interval were considered active. The area under the blood glucose curve over the 2 hour period after the administration of the glucose was calculated for each compound and compared with the value for control animals.

| Compounds of Example No. | Dose µmol/kg | % Reduction in area under blood glucose curve |
|---|---|---|
| 2 | 2.5 | 22 |
| 3 | 2.5 | 3 |
| 6 | 10 | 29 |
| 7 | 25 | 11 |

4. Assessment of Inotropic Activity

a) Activity of compounds on isolated rat atria

Sprague-Dawley male rats (300-400g) are killed and the right and left atria dissected out independently and mounted on a combined tissue holder/electrode. The tissues are then immersed in a glass bath containing Krebs solution maintained at 32°C. The rate of the spontaneously beating right atrium (via an isometric transducer and a ratemeter) and the tension of the electrically paced left atrium (via an isometric transducer) are recorded on a M19 chart recorder.

After an initial stabilization period, the tissues are exposed to a maximal concentration of isoprenaline ($10^{-7}$M). The tissues are then washed several times until rate and tension return to baseline level. A dose-response curve to the test compound is then carried out.

Responses to each concentration of test compound are expressed as a percentage of the maximal responses to isoprenaline. Results are given in the form of (a) intrinsic activity (maximal effect of test compound with respect to isoprenaline maximum, i.e. isoprenaline intrinsic activity = 1) and (b) $EC_{50}$ (the molar concentration at which the test compound produces 50% of its own maximum response).

cont..

0171702

| Compound of Example No. | Rate Intrinsic Activity | $EC_{50}$ | Tension Intrinsic Activity | $EC_{50}$ |
|---|---|---|---|---|
| 1 | 0.97 | $6.2 \times 10^{-9}$ | 0.97 | $2.1 \times 10^{-8}$ |
| 2 | 0.44 | $5.5 \times 10^{-8}$ | 0.18 | $8.5 \times 10^{-8}$ |
| 3 | 0.72 | $5.5 \times 10^{-9}$ | 0.52 | $2.1 \times 10^{-8}$ |
| 4 | 0.89 | $1.4 \times 10^{-8}$ | 0.4 | $5 \times 10^{-8}$ |
| 5 | 0.18 | $3.5 \times 10^{-8}$ | - | - |
| 6 | 0.66 | $2.5 \times 10^{-8}$ | 0.07 | $3 \times 10^{-7}$ |
| 7 | 0.5 | $3.6 \times 10^{-8}$ | 0.14 | $4.5 \times 10^{-8}$ |
| 8 (14:86) | 0.16 | $7.5 \times 10^{-7}$ | 0.20 | $3.8 \times 10^{-6}$ |
| 8 (82:18) | 0.29 | $2.4 \times 10^{-7}$ | 0.12 | $3.2 \times 10^{-6}$ |
| 9 | 0.15 | $3 \times 10^{-8}$ | 0.12 | $2.8 \times 10^{-8}$ |
| 11 | 0.32 | $5.3 \times 10^{-8}$ | 0.11 | $1 \times 10^{-7}$ |
| 14 | 0.87 | $4 \times 10^{-7}$ | 0.54 | $1.4 \times 10^{-6}$ |
| 15 | 0.98 | $4.4 \times 10^{-8}$ | 0.88 | $1.6 \times 10^{-7}$ |
| 18 | 0.52 | $1.1 \times 10^{-8}$ | - | - |

The following results have been obtained from experiments where the tissues were immersed in an organ bath at 29°C.

| | | | | |
|---|---|---|---|---|
| 12 | 0.98 | $2.9 \times 10^{-8}$ | 0.9 | $7.9 \times 10^{-7}$ |
| 13 | 0.64 | $2.5 \times 10^{-7}$ | 0.54 | $1.5 \times 10^{-5}$ |
| 17 | 0.91 | $8.6 \times 10^{-9}$ | - | - |
| 19 | 0.54 | $1.3 \times 10^{-7}$ | - | - |
| 20 | 0.95 | $6.3 \times 10^{-8}$ | 1.13 | $1.3 \times 10^{-7}$ |
| 21 | 0.85 | $1.6 \times 10^{-8}$ | - | - |

b) Assessment of inotropic activity in conscious cats

Male 3-4.5 kg cats, bred in-house are implanted with a polythene cannula in the left ventricle which is exteriorised at the back of the neck by means of a small teflon valve. Recordings are made via a pressure transducer attached by means of a length of polyethylene tubing to the cat neck valve. The amplified left ventricular pressure (LVP) signal is displayed on a chart recorder and from this signal measurements of heart rate (HR) are obtained via a ratemeter and rate of change (dp/dt) of left ventricular pressure (LVP) is obtained via a differentiator.

During an initial 1 hour stabilisation period, steady baseline recordings are obtained. The test compound contained in a gelatine capsule, is then administered orally. Recording is normally carried out for 6 hours post dosing.

The maximal percentage change in dp/dt over initial baseline level is used as the index of inotropic activity. Chronotropic activity is taken as the maximal percentage change in HR over the initial baseline level.

| Compound of Example No. | Dose mg/kg p.o. | Peak dp/dt | Peak HR | dp/dt: HR |
|---|---|---|---|---|
| 1 | 1 | 230 | 46 | 5.0 |
| 3 | 0.2 | 23 | 0 | - |
| 3 | 1 | 95 | 13 | 7.3 |
| 4 | 1 | 50 | 36 | 1.4 |

CLAIMS C

1. An unsubstituted or substituted (aryl- or heterocyclyl- or aryloxy- or heterocyclyloxy-) $C_{2-3}$-alkanolamino- ($C_{2-4}$alkyl- or $C_{2-3}$alkoxy)-benzoxazinone or a pharmaceutically acceptable salt thereof or ester thereof.

2. A compound as claimed in claim 1, of general formula (A):

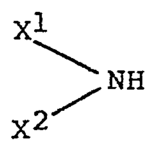

(A)

or a pharmaceutically acceptable salt thereof, wherein: $X^1$ represents a 2-hydroxy-3-phenoxypropyl radical

unsubstituted or substituted in the phenyl ring, or a 2-hydroxy-2-phenethyl radical unsubstituted or substituted in the phenyl ring or a 2-hydroxy-2-(benzofuranyl)ethyl radical or a 2-hydroxy-3-(indolyl)oxypropyl radical wherein a phenyl moiety in either of the benzofuranyl and indolyl moieties may be substituted or unsubstituted, and

$X^2$ represents a benzoxazinonylethyl,

benzoxazinonylpropyl or benzoxazinonyloxyethyl radical unsubstituted or substituted in the benzoxazinonyl radical and/or lower alkyl-substituted in the alkyl chain.

3. A compound as claimed in claim 1 or claim 2, of the general formula (I):

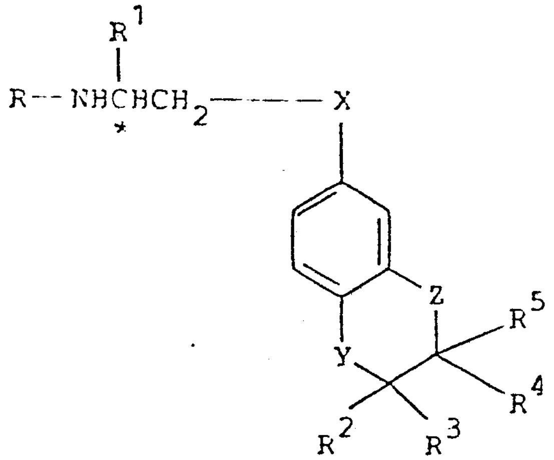

(I)

in which

R represents a 2-hydroxy-2-(benzofuranyl)ethyl moiety or a 2-hydroxy-2-(indolyl)oxypropyl moiety wherein the phenyl group in either of the two moieties may be substituted or unsubstituted; or a radical of the general formula (a) or (b):

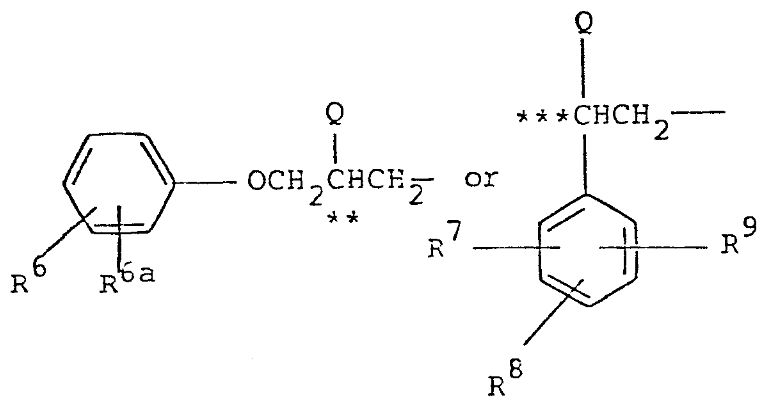

or

(a) (b)

$R^1$ represents a hydrogen atom or a lower alkyl group,
$R^6$ and $R^{6a}$ each represent a hydrogen atom or a free hydroxy group or an ester thereof
or a lower-alkyloxy group or a lower-alkenyloxy group,
$R^7$ represents a hydrogen or halogen atom, a hydroxy group or an ester thereof or an
ether thereof, or a group $CF_3$, $CH_2OH$ or an

ester thereof, $NH_2$, $NHR^{11}$, $NHCOOR^{11}$, or $CH_2SO_2R^{11}$ in which $R^{11}$ represents an alkyl radical, $R^8$ represents a hydrogen or halogen atom, or a hydroxy group or an ester thereof or an ether thereof,

$R^9$ represents a hydrogen or halogen atom,
Q represents a hydroxy group or an <u>in-vivo</u> hydrolysable ester thereof, X represents a bond, a methylene group or an oxygen atom, and
Y represents an oxygen atom or a radical $NR^{10}$ in which $R^{10}$ represents a hydrogen atom or an alkyl group, and
Z represents an oxygen atom or a radical $NR^{10}$ with the proviso that
when Y represents an oxygen atom, Z represents a radical $NR^{10}$, $R^2$ and $R^3$ each represents a hydrogen atom and $R^4$ and $R^5$ together represent an oxo group
or
when Y represents a radical $NR^{10}$, Z represents an oxygen atom, $R^2$ and $R^3$ together represent an oxo group and $R^4$ and $R^5$ each represents a hydrogen atom.

4. A compound as claimed in claim 3, wherein the moiety of formula (a) is one of the following groups

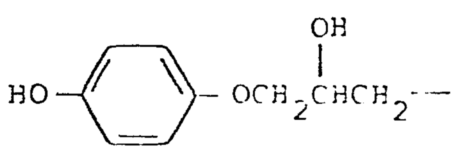 , 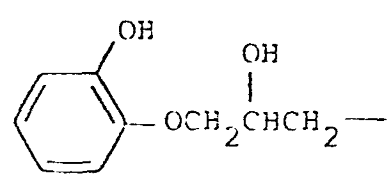

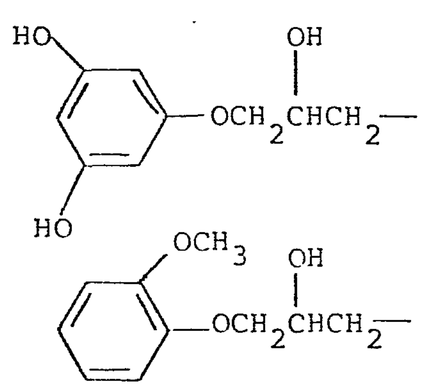 , 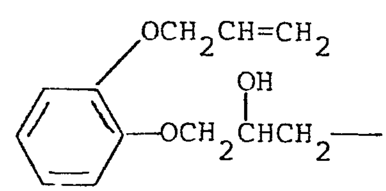

, 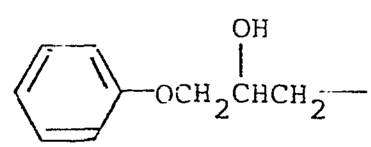

5. A compound as claimed in claim 3, wherein the radical of formula (b) is one of the following groups:

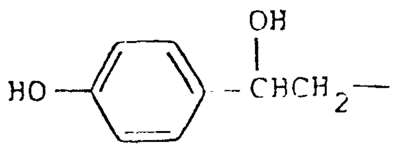 , 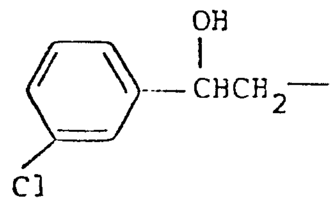

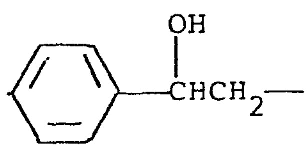 , 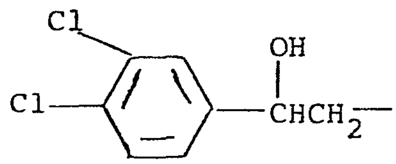

6. A compound as claimed in claim 1, selected from the group consisting of:

7-[2-[2-(4-hydroxyphenyl)-2-hydroxyethylamino]-2-methylethoxy]-2H-1,4-benzoxazin-3-(4H)-one;

7-[2-[2-(3-chlorophenyl)-2-hydroxyethylamino]-2-methylethoxy]-2H-1,4-benzoxazin-3-(4H)-one;

7-[2-[3-(4-hydroxyphenoxy)-2-hydroxypropylamino]-2-methylethoxy]-2H-1,4-benzoxazin-3-(4H)-one;

6-[2-[3-(4-hydroxyphenoxy)-2-hydroxypropylamino]-2-methylethoxy]-2H-1,4-benzoaxazin-3-(4H)-one;

6-[2-[3-(2-hydroxyphenoxy)-2-hydroxypropylamino]-2-methylethoxy]-2H-1,4-benzoxazin-3-(4H)-one;

6-[2-[2-(3-chlorophenyl)-2-hydroxyethylamino]-2-methylethoxy]-2H-1,4-benzoxazin-3-(4H)-one;

6-[2-[2-(3,4-dichlorophenyl)-2-hydroxyethylamino]-2-methylethoxy]-2H-1,4-benzoxazin-3-(4H)-one

6-[2-[2-(3-chlorophenyl)-2-hydroxyethylamino]-2-methylethyl]-2H-1,4-benzoxazin-3-(4H)-one;

6-[2-[3-(2-hydroxyphenoxy)-2-hydroxypropylamino]-2-methylethyl]-2-H-1,4-benzoxazin-3-(4H)-one;

6-[2-[2-phenyl-2-hydroxyethylamino]-2-methylethyl]-2H-1,4-benzoxazin-3-(4H)-one;

6-[2-[3-phenoxy-2-hydroxypropylamino]-2-methylethyl]-2H-1,4-benzoxazin-3-(4H)-one;

6-[2-[2-(3-chlorophenyl)-2-hydroxyethylamino]-2-methylethoxy]-4-methyl-2H-1,4-benzoxazin-3-(4H)-one;

6-[2-[2-(3-chlorophenyl)-2-hydroxyethylamino]-2-methylethyl]-4-methyl]-2H-1,4-benzoxazin-3-(4H)-one;

6-[2-[3-(4-hydroxyphenoxy)-2-hydroxypropylamino]-2-methylethyl]-2H-1,4-benzoxazin-3-(4H)-one;

6-[2-[2-(4-hydroxyphenyl)-2-hydroxyethylamino]-2-methylethoxy]-2H-1,4-benzoxazin-3-(4H)-one;

6-[2-[3-(2-allyloxyphenoxy)-2-hydroxypropylamino]-2-methylethoxy]-2H-1,4-benzoxazin-3-(4H)-one;

6-[2-[3-(2-methoxyphenoxy)-2-hydroxypropylamino]-2-methylethoxy]-2H-1,4-benzoxazin-3-(4H)-one;

6-[2-(2-hydroxy-3-phenoxypropylamino)-2-methylethoxy) -2H-1,4-benzoxazin-3-(4H)-one;

6-[2-[2-hydroxy-3-(indol-4-yloxy)propylamino]-2-methylethoxy]-2H-1,4-benzoxazin-3-(4H)-one;

6-[2-[2-(7-ethylbenzofuran-2-yl)-2-hydroxyethylamino] -2-methylethoxy]-2H-1,4-benzoxazin-3-(4H)-one; and

6-[2-[3-(3,5-dihydroxyphenoxy)-2-hydroxypropylamino]-2-methylethoxy-2H-1,4-benzoxazin-3-(4H)-one; or a pharmaceutically acceptable salt thereof.

7. A process for the preparation of a compound of formula (A), or a pharmaceutically acceptable salt thereof, which process comprises: either

(a) reacting a compound of formula (B):

$$Y^1NH_2 \qquad (B):$$

wherein $Y^1$ represents either $X^1$ or $X^2$, and wherein $X^1$ and $X^2$ are as defined in relation to formula (A), with:

(i) a compound of formula (C):

$$Y^2\text{-}A \qquad (C):$$

wherein $Y^2$ represents either $X^1$ or $X^2$, as defined above, but does not represent $Y^1$, and A represents a leaving group; or

(ii) a compound of formula (D):

$$Y^3\text{-}A \qquad (D):$$

wherein $Y^3$ represents a group which is convertible, preferably by reduction, to a group of formula $Y^2$ after attachment to the nitrogen atom of formula (B) and A represents a leaving group; or

(b) reacting a compound of formula (E):

$$X^2NH_2 \qquad (E):$$

wherein $X^2$ is as defined above, with a compound of formula (F):

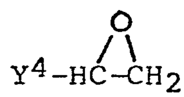

(F):

wherein $Y^4$ represents a group which when present in the moiety 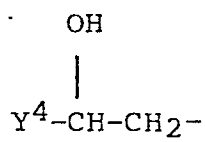 the moiety represents $X^1$, or

(c) by reducing a compound of formula (G):

$$Y^4\text{-CO-C=N-}X^2 \qquad (G):$$

wherein $Y^4$ and $X^2$ are as defined above;
and thereafter, if necessary, carrying out one or more of the following steps:

(a) converting a compound of formula (A) to another compound of formula (A);

(b) converting any hydroxy group to an ester group thereof;

and if required, thereafter, forming a pharmaceutically acceptable salt of the compound so formed.

8. A process for the preparation of a compound of the general formula (I) or a pharmaceutically acceptable salt thereof, which comprises:

A) reacting a compound of the general formula (II):

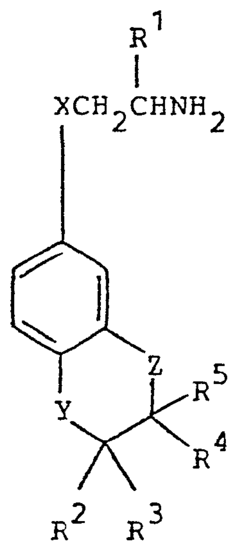

(II)

in which $R^1$, $R^2$, $R^3$, $R^4$, $R^5$, X, Y and Z are as defined in relation to formula (I) or a salt or reactive derivative thereof, with

(i) a compound of the general formula (III):

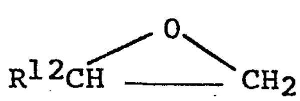

(III)

in which $R^{12}$ represents a benzofuranyl moiety or an indolyloxymethylene moiety wherein the phenyl group in

either of the two moities may be substituted or unsubstituted, or $R^{12}$ represents a group of the general formula:

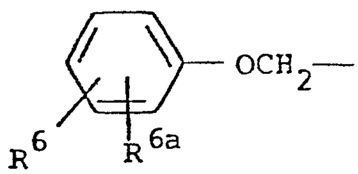

or

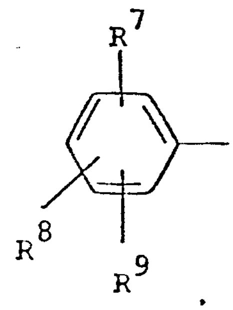

in which $R^6$, $R^{6a}$, $R^7$, $R^8$ and $R^9$ are as defined in relation to formula (I), or

(ii) a compound of the general formula (IV):

$R^{12}CHCH_2A$ (IV)

|

OH

in which $R^{12}$ has the meaning given above and A represents a leaving group as hereinafter defined, or an ester thereof; or

B) reacting a compound of the general formula (II) specified above, or a salt or reactive derivative thereof, with

(i) a compound of the general formula (V):

$R^{12}COCHO$ (V)

in which $R^{12}$ is as defined in relation to formula (III), and subsequently reducing the resulting compound of the general formula (VI):

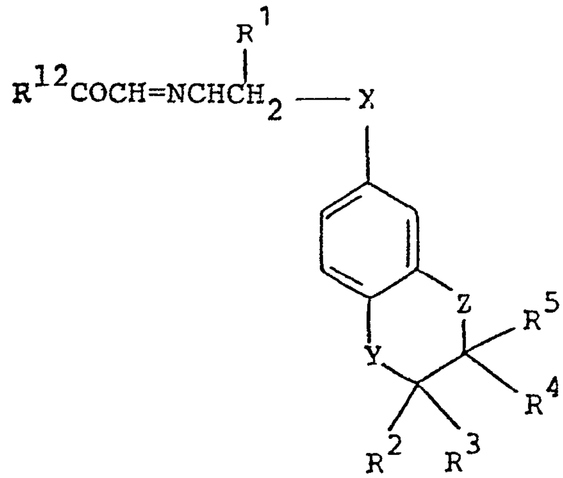

(VI)

in which $R^1$, $R^2$, $R^3$, $R^4$, $R^5$, X, Y and Z are as defined in relation to formula (I) and $R^{12}$ is as defined in relation to formula (III), or a salt thereof;
or

(ii) a compound of the general formula (VII):

$$R^{12}COCH_2A \qquad (VII)$$

in which $R^{12}$ and A have the meanings given above, and subsequently reducing the resulting compound of the general formula (VI'):

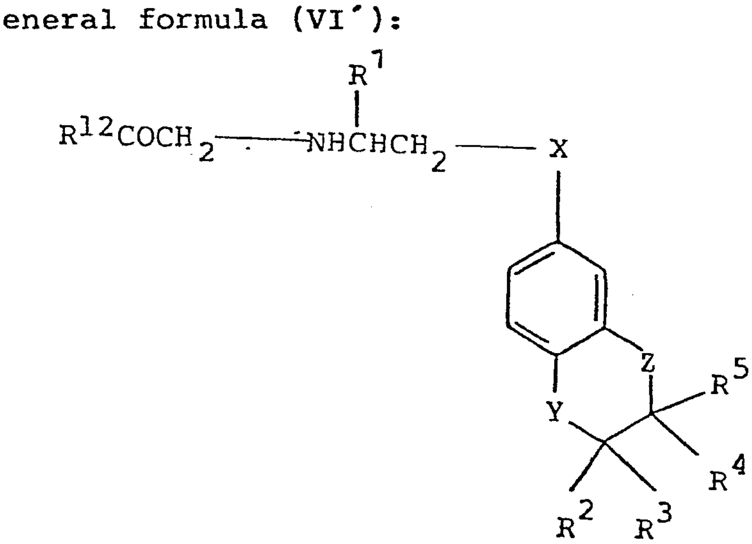

(VI')

in which $R^1$, $R^2$, $R^3$, $R^4$, $R^5$, X and Y and Z are as defined in relation to formula (I) and $R^{12}$ is as defined in relation to formula (III), or a salt thereof; or

C) reacting a compound of the general formula (VIII):

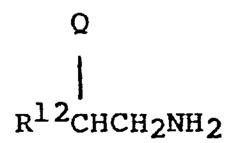

(VIII)

in which $R^{12}$ is as defined in relation to formula (III), and Q is as defined in relation to formula (I), or a salt or reactive derivative thereof, with

(i) a compound of the general formula (IX):

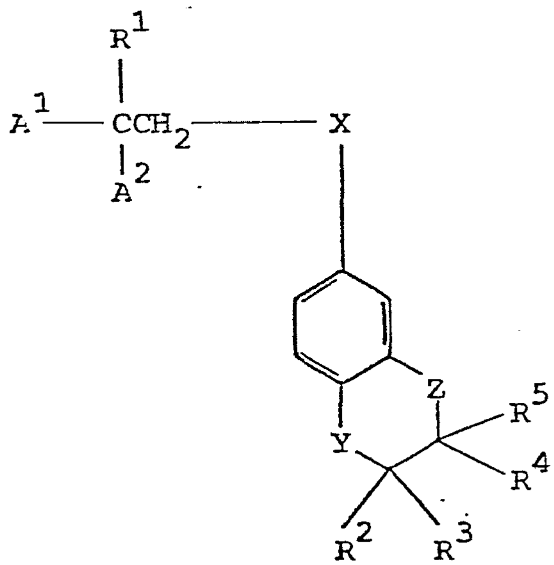

(IX)

in which $R^1$, $R^2$, $R^3$, $R^4$, $R^5$, X and Y and Z are as defined in relation to formula (I), $A^1$ represents a leaving group and $A^2$ represents a hydrogen atom, or

(ii) a compound of the general formula (IX) in which $R^1$, $R^2$, $R^3$, $R^4$, $R^5$, X and Y and Z have the meanings given above and $A^1$ and $A^2$ together represent an oxo group, and subsequently treating the intermediate with a reducing agent;

and, if desired, converting a resulting compound of the general formula (I) into another compound of the general formula (I) or into a pharmaceutically acceptable salt thereof.

9. A compound as claimed in claim 1, or a pharmaceutically acceptable salt thereof, for use as an active therapeutic substance.

10 A compound as claimed in claim 1, or a pharmaceutically acceptable salt thereof, for use in the treatment of cardiac disease or congestive heart failure in the human or non-human animal body.

11. A compound as claimed in claim 1, or a pharmaceutically acceptable salt thereof, for use in the treatment of hyperglycaemia and/or obesity in the human or non-human animal body.

12. A pharmaceutical composition comprising a compound as claimed in claim 1, or a pharmaceutically acceptable salt thereof, in admixture or conjunction with a pharmaceutically acceptable carrier therefore.

13. A method for improving the weight gain and/or improving the feed utilisation efficiency and/or increasing lean body mass of livestock, which method comprises the administration to livestock of an effective non-toxic amount of a compound as claimed in claim 1, or a veterinarily acceptable salt thereof.

European Patent Office

0171702

Application number

# EUROPEAN SEARCH REPORT

EP 85 10 9577

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| A | GB-A-1 549 945 (CIBA-GEIGY) * Claims * | 1,7-13 | C 07 D 265/36<br>C 07 D 413/12<br>A 61 K 31/535 |
| | --- | | |
| A | GB-A-1 592 975 (BOEHRINGER) * Claims 1,46-88 * | 1,7-13 | |
| | --- | | |
| D,A | US-A-4 349 673 (J.G. ATKINSON) * Column 12, line 6; example 2; claims * | 1,7 | |
| | ----- | | |
| | | | TECHNICAL FIELDS SEARCHED (Int. Cl 4) |
| | | | C 07 D 265/00<br>C 07 D 413/00<br>A 61 K 31/00 |

The present search report has been drawn up for all claims

| Place of search | Date of completion of the search | Examiner |
|---|---|---|
| THE HAGUE | 06-11-1985 | CHOULY J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document